# EUROPEAN PATENT APPLICATION

(11) **EP 1 702 916 A1**
(43) Date of publication of application: **20.09.2006**
(21) Application number: 05006015.1
(22) Date of filing: 18.03.2005
(51) Int. Cl.: C07D 211/16, C07D 211/22, C07D 211/58, C07D 211/34, C07D 401/04, C07D 211/76, C07D 211/46, C07D 207/06, C07D 401/06, C07D 413/14, C07D 207/08, C07D 413/04, C07D 211/18, C07D 211/38, C07D 211/42, C07D 211/54, C07D 205/04, C07D 401/12, C07D 207/14, C07D 207/09, C07D 207/48, A61K 31/40, A61K 31/397, A61K 31/445, A61P 3/04, A61P 3/10

(54) **DPP-IV inhibitors**

(71) Applicant: Santhera Pharmaceuticals (Schweiz) GmbH, 4410 Liestal (CH)
(72) Inventor: Edwards, Paul John, 69115 Heidelberg (DE); Cerezo-Galvez, Silvia, 42287 Wuppertal (DE); Lopez-Canet, Meritxell, 69123 Heidelberg (DE); Matassa, Victor Giulio, La Floresta Barcelona 08198 (ES); Nordhoff, Sonja, 69198 Schriesheim (DE); Rosenbaum, Claudia, 50354 Hürth (DE); Rummey, christian, 69221 Dossenheim (DE)
(74) Representative: Grünecker, Kinkeldey, Stockmair & Schwanhäusser Anwaltssozietät

(57) **Abstract**

The invention relates to compounds of formula (I) 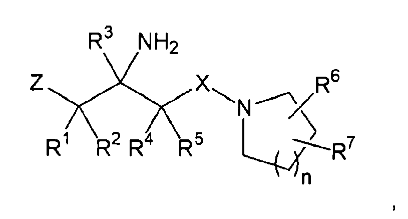 wherein Z, R¹⁻⁷, X and n have the meaning as cited in the description and the claims. Said compounds are useful as DPP-IV inhibitors. The invention also relates to the preparation of such compounds as well as the production and use thereof as medicament.

## Description

The present invention relates to a novel class of dipeptidyl peptidase inhibitors, including pharmaceutically acceptable salts and prodrugs thereof, which are useful as therapeutic compounds, particularly in the treatment of Type 2 diabetes mellitus, often referred to as non-insulin dependent diabetes mellitus (NIDDM), and of conditions that are often associated with this disease, such as obesity and lipid disorders.

Diabetes refers to a disease process derived from multiple causative factors and characterized by elevated levels of plasma glucose or hyperglycemia in the fasting state or after administration of glucose during an oral glucose tolerance test. Persistent or uncontrolled hyperglycemia is associated with increased and premature morbidity and mortality. Often abnormal glucose homeostasis is associated both directly and indirectly with alterations of the lipid, lipoprotein and apolipoprotein metabolism and other metabolic and hemodynamic disease. Therefore patients with Type 2 diabetes mellitus are at an increased risk of macrovascular and microvascular complications, including coronary heart disease, stroke, peripheral vascular disease, hypertension, nephropathy, neuropathy, and retinopathy. Therefore, therapeutic control of glucose homeostasis, lipid metabolism and hypertension are critically important in the clinical management and treatment of diabetes mellitus.

There are two generally recognized forms of diabetes. In Type 1, or insulin-dependent, diabetes mellitus (IDDM), patients produce little or no insulin, which is the hormone regulating glucose utilization. In Type 2, or noninsulin dependent, diabetes mellitus (NIDDM), patients often have plasma insulin levels that are the same or elevated compared to nondiabetic subjects. These patients develop a resistance to the insulin stimulating effect on glucose and lipid metabolism in the main insulin-sensitive tissues, namely the muscle, liver and adipose tissues. Further, the plasma insulin levels, while elevated, are insufficient to overcome the pronounced insulin resistance.

Insulin resistance is not primarily due to a diminished number of insulin receptors but to a post-insulin receptor binding defect that is not yet understood. This resistance to insulin responsiveness results in insufficient insulin activation of glucose uptake, oxidation and storage in muscle, and inadequate insulin repression of lipolysis in adipose tissue and of glucose production and secretion in the liver.

The available treatments for Type 2 diabetes, which have not changed substantially in many years, have recognized limitations. While physical exercise and reductions in dietary intake of calories will dramatically improve the diabetic condition, compliance with this treatment is very poor because of well-entrenched sedentary lifestyles and excess food consumption, especially of foods containing high amounts of saturated fat. Increasing the plasma level of insulin by administration of sulfonylureas (e.g., tolbutamide and glipizide) or meglitinide, which stimulate the pancreatic β-cells to secrete more insulin, and/or by injection of insulin when sulfonylureas or meglitinide become ineffective, can result in insulin concentrations high enough to stimulate the very insulin-resistant tissues. However, dangerously low levels of plasma glucose can result from administration of insulin or insulin secretagogues (sulfonylureas or meglitinide), and an increased level of insulin resistance, due to the even higher plasma insulin levels, can occur. The biguanides increase insulin sensitivity resulting in some correction of hyperglycemia. However, the two biguanides, phenformin and metformin, can induce lactic acidosis and nausea/diarrhoea. Metformin has fewer side effects than phenformin and is often prescribed for the treatment of Type 2 diabetes.

The glitazones (*i.e.*, 5-benzylthiazolidine-2,4-diones) are a recently described class of compounds with potential for ameliorating many symptoms of Type 2 diabetes. These agents substantially increase insulin sensitivity in muscle, liver and adipose tissue in several animal models of Type 2 diabetes, resulting in partial or complete correction of the elevated plasma levels of glucose without occurrence of hypoglycemia. The glitazones that are currently marketed are agonists of the peroxisome proliferator activated receptor (PPAR), primarily the PPAR-gamma subtype. PPAR-gamma agonism is generally believed to be responsible for the improved insulin sensitization that is observed with the glitazones. Newer PPAR agonists that are being tested for treatment of Type 2 diabetes are agonists of the alpha, gamma or delta subtype, or a combination of these, and in many cases are chemically different from the glitazones (*i*.*e*., they are not thiazolidinediones). Serious side effects (e.g., liver toxicity) have occurred with some of the glitazones, such as troglitazone.

Additional methods of treating the disease are still under investigation. New biochemical approaches that have been recently introduced or are still under development include treatment with alpha-glucosidase inhibitors (e.g., acarbose) and protein tyrosine phosphatase-IB (PTP-1 B) inhibitors.

Compounds that are inhibitors of the dipeptidyl peptidase-IV (DPP-IV) enzyme are also under investigation as drugs that may be useful in the treatment of diabetes, and particularly Type 2 diabetes. See for example WO-A-97/40832, WO-A-98/19998, WO-A-03/180 and WO-A-03/181. The usefulness of DPP-IV inhibitors in the treatment of Type 2 diabetes is based on the fact that DPP-IV *in vivo* readily inactivates glucagon like peptide-1 (GLP-1) and gastric inhibitory peptide (GIP). GLP-1 and GIP are incretins and are produced when food is consumed. The incretins stimulate production of insulin. Inhibition of DPP-IV leads to decreased inactivation of the incretins, and this in turn results in increased effectiveness of the incretins in stimulating production of insulin by the pancreas. DPP-IV inhibition therefore results in an increased level of serum insulin. Advantageously, since the incretins are produced by the body only when food is consumed, DPP-IV inhibition is not expected to increase the level of insulin at inappropriate times, such as between meals, which can lead to excessively low blood sugar (hypoglycemia). Inhibition of DPP-IV is therefore expected to increase insulin without increasing the risk of hypoglycemia, which is a dangerous side effect associated with the use of insulin secretagogues.

DPP-IV inhibitors may also have other therapeutic utilities, as discussed elsewhere in this application. DPP-IV inhibitors have not been studied extensively to date, especially for utilities other than diabetes. New compounds are needed so that improved DPP-IV inhibitors can be found for the treatment of diabetes and potentially other diseases and conditions.

Thus, the object of the present invention is to provide a new class of DPP-IV inhibitors which may be effective in the treatment of Type 2 diabetes and other DPP-IV modulated diseases.

Accordingly, the present invention provides novel compounds of formula (I): 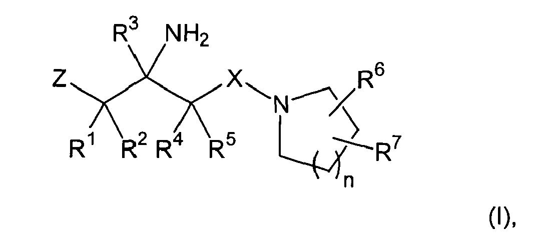 or a pharmaceutically acceptable salt or prodrug thereof, wherein
Z is selected from the group consisting of phenyl; naphthyl; indenyl; C₃₋₇ cycloalkyl; indanyl; tetralinyl; decalinyl; heterocycle; and heterobicycle, wherein Z is optionally substituted with one or more R⁸, wherein R⁸ is independently selected from the group consisting of halogen; CN; OH; NH₂; oxo (=O), where the ring is at least partially saturated; R⁹; and R¹⁰;
R⁹ is selected from the group consisting of C₁₋₆ alkyl; O-C₁₋₆ alkyl; and S-C₁₋₆ alkyl, wherein R⁹ is optionally interrupted by oxygen and wherein R⁹ is optionally substituted with one or more halogen independently selected from the group consisting of F; and Cl;
R¹⁰ is selected from the group consisting of phenyl; heterocycle; and C₃₋₇ cycloalkyl, wherein R¹⁰ is optionally substituted with one or more R¹¹, wherein R¹¹ is independently selected from the group consisting of halogen; CN; OH; NH₂; oxo (=O), where the ring is at least partially saturated; C₁₋₆ alkyl; O-C₁₋₆ alkyl; and S-C₁₋₆ alkyl;
R¹, R⁴ are independently selected from the group consisting of H; F; OH; and R^{4a};
R², R⁵ are independently selected from the group consisting of H; F; and R^{4b};
R^{4a} is independently selected from the group consisting of C₁₋₆ alkyl; and O-C₁₋₆ alkyl, wherein R^{4a} is optionally substituted with one or more halogen independently selected from the group consisting of F; and Cl;
R^{4b} is C₁₋₆ alkyl, wherein R^{4b} is optionally substituted with one or more halogen independently selected from the group consisting of F; and Cl;
R³ is selected from the group consisting of H; and C₁₋₆ alkyl;

Optionally one or more pairs of R¹, R², R³, R⁴, R⁵ independently selected from the group consisting of R¹/R²; R²/R³; R³/R⁴; and R⁴/R⁵ form a C₃₋₇ cycloalkyl ring, which is optionally substituted with one or more of R¹², wherein R¹² is independently selected from the group consisting of F; Cl; and OH;
n is 0, 1 or 2;
X is selected from the group consisting of S(O); S(O)₂; C(O); and C(R¹³R¹⁴);
R¹³, R¹⁴ are independently selected from the group consisting of H; F; C₁₋₆ alkyl; R¹⁵; and R¹⁶;
Optionally one or both pairs of R⁵, R¹³, R¹⁴ selected from the group consisting of R⁵/R¹³; and R¹³/R¹⁴ form a C₃₋₇ cycloalkyl ring, which is optionally substituted with one or more R¹⁷, wherein R¹⁷ is independently selected from the group consisting of F; Cl; and OH;
R¹⁵ is selected from the group consisting of phenyl; naphthyl; and indenyl, wherein R¹⁵ is optionally substituted with one or more R¹⁸, wherein R¹⁸ is independently selected from the group consisting of R¹⁹; R²⁰; halogen; CN; COOH; OH; C(O)NH₂; S(O)₂NH₂; S(O)NH₂; C₁₋₆ alkyl; O-C₁₋₆ alkyl; S-C₁₋₆ alkyl; COO-C₁₋₆ alkyl; OC(O)-C₁₋₆ alkyl; C(O)N(R²¹)-C₁₋₆ alkyl; S(O)₂N(R²¹)-C₁₋₆ alkyl; S(O)N(R²¹)-C₁₋₆ alkyl; S(O)₂-C₁₋₆ alkyl; S(O)-C₁₋₆ alkyl; N(R²¹)S(O)₂-C_{1- 6} alkyl; and N(R²¹)S(O)-C₁₋₆ alkyl, wherein each C₁₋₆ alkyl is optionally substituted with one or more halogen independently selected from the group consisting of F; and Cl;
R¹⁶ is selected from the group consisting of heterocycle; heterobicycle; C₃₋₇ cycloalkyl; indanyl; tertralinyl; and decalinyl, wherein R¹⁶ is optionally substituted with one or more R²², wherein R²² is independently selected from the group consisting of R¹⁹; R²⁰; halogen; CN; OH; oxo (=O), where the ring is at least partially saturated; NH₂; COOH; C(O)NH₂; S(O)₂NH₂; S(O)NH₂; C₁₋₆ alkyl; O-C₁₋₆ alkyl; S-C₁₋₆ alkyl; N(R²³)-C₁₋₆ alkyl; COO-C₁₋₆ alkyl; OC(O)-C₁₋₆ alkyl; C(O)N(R²³)- C₁₋₆ alkyl; N(R²³)-C(O)-C₁₋₆ alkyl; S(O)₂N(R²³)-C₁₋₆ alkyl; S(O)N(R²³)-C₁₋₆ alkyl; S(O)₂-C₁₋₆ alkyl; S(O)-C₁₋₆ alkyl; N(R²³)S(O)₂-C₁₋₆ alkyl; and N(R²³)S(O)-C₁₋₆ alkyl, wherein each C₁₋₆ alkyl is optionally substituted with one or more halogen independently selected from the group consisting of F; and Cl;
R¹⁹ is selected from the group consisting of phenyl; and naphthyl, wherein R¹⁹ is optionally substituted with one or more R²⁴, wherein R²⁴ is independently selected from the group consisting of halogen; CN; COOH; OH; C(O)NH₂; S(O)₂NH₂; S(O)NH₂; C₁₋₆ alkyl; O-C₁₋₆ alkyl; S-C₁₋₆ alkyl; COO-C₁₋₆ alkyl; OC(O)-C₁₋₆ alkyl; C(O)N(R²⁵)-C₁₋₆ alkyl; S(O)₂N(R²⁵)-C₁₋₆ alkyl; S(O)N(R²⁵)-C₁₋₆ alkyl; S(O)₂-C₁₋₆ alkyl; S(O)-C₁₋₆ alkyl; N(R²⁵)S(O)₂-C₁₋₆ alkyl; and N(R²⁵)S(O) -C₁₋₆ alkyl, wherein each C₁₋₆ alkyl is optionally substituted with one or more halogen independently selected from the group consisting of F; and Cl;
R²⁰ is selected from the group consisting of heterocycle; heterobicycle; and C₃₋₇ cycloalkyl; wherein R²⁰ is optionally substituted with one or more R²⁶, wherein R²⁶ is independently selected from the group consisting of halogen; CN; OH; oxo (=O), where the ring is at least partially saturated; NH₂; COOH; C(O)NH₂; S(O)₂NH₂; S(O)NH₂; C₁₋₆ alkyl; O-C₁₋₆ alkyl; S-C₁₋₆ alkyl; N(R²⁷)-C₁₋₆ alkyl; COO-C₁₋₆ alkyl; OC(O)-C₁₋₆ alkyl; C(O)N(R²⁷)- C₁₋₆ alkyl; N(R²⁷)-C(O)-C₁₋₆ alkyl; S(O)₂N(R²⁷)-C₁₋₆ alkyl; S(O)N(R²⁷)-C₁₋₆ alkyl; S(O)₂-C₁₋₆ alkyl; S(O)-C₁₋₆ alkyl; N(R²⁷)S(O)₂-C₁₋₆ alkyl; and N(R²⁷)S(O)-C₁₋₆ alkyl wherein each C₁₋₆ alkyl is optionally substituted with one or more halogen independently selected from the group consisting of F; and Cl;
R²¹, R²³, R²⁵, R²⁷ are independently selected from the group consisting of H; and C₁₋₆alkyl, which is optionally substituted with one or more of R²⁸, wherein R²⁸ is independently selected from the group consisting of F; Cl and OH;
R⁶ is selected from the group consisting of
(1) halogen; CN; OH; NH₂; COOH; C(O)NH₂; S(O)₂NH₂; S(O)NH₂; C₁₋₆ alkyl; O-C₁₋₆ alkyl; N(R^{32a})-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆ alkyl-T³; C(O)N(R^{32a})-T³; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂₋T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl; ₁₋₆ alkyl-T³; OT³; wherein each C₁₋₆ alkyl is optionally substituted with one or more OH or halogen independently selected from the group consisting of F; and Cl; with the proviso that when X is C(O), R⁶ is not (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂₋T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl;
(2) phenyl; naphthyl; and indenyl; which are each optionally substituted with one or more R³⁸; wherein R³⁸ is independently selected from the group consisting of halogen; CN; R³⁹; COOH; OH; C(O)NH₂; S(O)₂NH₂; S(O)NH₂; COOT³; OT³; ST³; C(O)N(R⁴⁰)T³; S(O)₂N(R⁴⁰)T³; S(O)N(R⁴⁰)T³ and T³;
(3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of halogen; CN; R⁴²; OH; oxo (=O), where the ring is at least partially saturated; NH₂; COOH; C(O)NH₂; S(O)₂NH₂; S(O)NH₂; COOT³; OT³; C(O)N(R⁴³)T³; S(O)₂N(R⁴³)T³; S(O)N(R⁴³)T³; N(R⁴³)T³; and T³;
R^{32a} is selected from the group consisting of H; C₃₋₇ cycloalkyl and C₁₋₆ alkyl, which is optionally substituted with one or more halogen independently selected from the group consisting of F; and Cl;
R³⁹ is selected from the group consisting of C₁₋₆ alkyl; O-C₁₋₆ alkyl; S-C₁₋₆ alkyl; COO-C₁₋₆ alkyl; OC(O)- C₁₋₆ alkyl; C(O)N(R⁴⁴)-C₁₋₆ alkyl; S(O)₂N(R⁴⁴)-C₁₋₆ alkyl; S(O)N(R⁴⁴)-C₁₋₆ alkyl; S(O)-C₁₋₆ alkyl; S(O)₂-C₁₋₆ alkyl; N(R⁴⁴)S(O)₂-C₁₋₆ alkyl; and N(R⁴⁴)S(O) -C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³;
R⁴² is selected from the group consisting of C₁₋₆ alkyl; O-C₁₋₆ alkyl; S-C₁₋₆ alkyl; N(R⁴⁸)-C₁₋₆ alkyl; COO-C₁₋₆ alkyl; OC(O)- C₁₋₆ alkyl; C(O)N(R⁴⁸)- C₁₋₆ alkyl; N(R⁴⁸)-C(O)-C₁₋₆ alkyl; S(O)₂N(R⁴⁸)-C₁₋₆ alkyl; S(O) N(R⁴⁸)-C₁₋₆ alkyl; S(O)-C₁₋₆ alkyl; S(O)₂-C₁₋₆ alkyl; - N(R⁴⁸)S(O)₂-C₁₋₆ alkyl; and -N(R⁴⁸)S(O)-C₁₋₆ alkyl; wherein each C₁₋₆ alkyl is optionally substituted with one or more R⁴⁵, wherein R⁴⁵ is independently selected from the group consisting of F; COOR⁴⁹; C(O)N(R⁴⁹R⁵⁰); S(O)₂N(R⁴⁹R⁵⁰); S(O)N(R⁴⁹R⁵⁰); OR⁴⁹; N(R⁴⁹R⁵⁰); T³; O-T³; and N(R⁴⁹)-T³;
R⁴⁰, R⁴³, R⁴⁴, R⁴⁶, R⁴⁷, R⁴⁸, R⁴⁹, R⁵⁰ are independently selected from the group consisting of H; and C₁₋₆ alkyl;
T³ is selected from the group consisting of T⁴; and T⁵;
T⁴ is selected from the group consisting of phenyl; naphthyl; and indenyl; wherein T⁴ is optionally substituted with one or more R⁵¹, wherein R⁵¹ is independently selected from the group consisting of halogen; CN; COOR⁵²; OR⁵²; C(O)N(R⁵²R⁵³); S(O)₂N(R⁵²R⁵³); C₁₋₆ alkyl; O-C₁₋₆ alkyl; S-C₁₋₆ alkyl; COO-C₁₋₆ alkyl; OC(O)-C₁₋₆ alkyl; C(O)N(R⁵²)- C₁₋₆ alkyl; S(O)₂N(R⁵²)-C₁₋₆ alkyl; S(O)N(R⁵²)-C₁₋₆ alkyl; S(O)₂-C₁₋₆ alkyl; S(O) -C₁₋₆ alkyl; N(R⁵²)S(O)₂-C₁₋₆ alkyl; and N(R⁵²)S(O)-C₁₋₆ alkyl; wherein each C₁₋₆ alkyl is optionally substituted with one more halogen selected from the group consisting of F; and Cl;
T⁵ is selected from the group consisting of heterocycle; heterobicycle; C₃₋₇ cycloalkyl; indanyl; tetralinyl; and decalinyl; wherein T⁵ is optionally substituted with one or more R⁵⁴, wherein R⁵⁴ is independently selected from the group consisting of halogen; CN; OR⁵⁵; oxo (=O), where the ring is at least partially saturated; N(R⁵⁵R⁵⁶); COOR⁵⁵; C(O)N(R⁵⁵R⁵⁶); S(O)₂N(R⁵⁵R⁵⁶); S(O)N(R⁵⁵R⁵⁶); C₁₋₆ alkyl; O-C₁₋₆ alkyl; S-C₁₋₆ alkyl; N(R⁵⁵)-C₁₋₆ alkyl; COO-C₁₋₆ alkyl; OC(O)- C₁₋₆ alkyl; C(O)N(R⁵⁵)- C₁₋₆ alkyl; N(R⁵⁵)-C(O)-C₁₋₆ alkyl; S(O)₂N(R⁵⁵)-C₁₋₆ alkyl; S(O)N(R⁵⁵)-C₁₋₆ alkyl; S(O)₂-C₁₋₆ alkyl; S(O)-C₁₋₆ alkyl; N(R⁵⁵)S(O)₂-C₁₋₆ alkyl; and N(R⁵⁵)S(O)-C₁₋₆ alkyl; wherein each C₁₋₆ alkyl is optionally substituted with one more halogen selected from the group consisting of F; and Cl;
R⁵², R⁵³, R⁵⁵, R⁵⁶, are independently selected from the group consisting of H; and C₁₋₆ alkyl; and
R⁷ is selected from the group consisting of hydrogen, halogen; CN; OH; NH₂; COOH; C(O)NH₂; S(O)₂NH₂; S(O)NH₂; or C₁₋₆ alkyl.

### Within the meaning of the present invention the terms are used as follows:

In case a variable or substituent can be selected from a group of different variants and such variable or substituent occurs more than once the respective variants can be the same or different.

"Alkyl" means a straight-chain or branched carbon chain that may contain double or triple bonds. It is generally preferred that alkyl doesn't contain double or triple bonds.

"C₁₋₄ Alkyl" means an alkyl chain having 1 - 4 carbon atoms, e.g. at the end of a molecule methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl or amid, e.g. -CH₂-, -CH₂₋CH₂-, -CH=CH-, -CH(CH₃)-, -C(CH₂)-, -CH₂-CH₂-CH₂-, -CH(C₂H₅)-, -CH(CH₃)₂-.

"C₁₋₆ Alkyl" means an alkyl chain having 1 - 6 carbon atoms, e.g. C₁₋₄ alkyl, methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, n-pentane, n-hexane, or amid, e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-.
Each hydrogen of a C₁₋₆ alkyl carbon may be replaced by a substituent.

"C₃₋₇ Cycloalkyl" or "C₃₋₇ Cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent.

"Halogen" means fluoro, chloro, bromo or iodo. It is generally preferred that halogen is fluoro or chloro.

"Heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine.

"Heterobicycle" means a heterocycle which is condensed with phenyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, dihydroquinoline, isoquinoline, tetrahydroisoquinoline, dihydroisoquinoline, benzazepine, purine or pteridine.

A preferred stereochemistry of compounds or a pharmaceutically acceptable salt thereof according to the present invention is shown in formula (la) 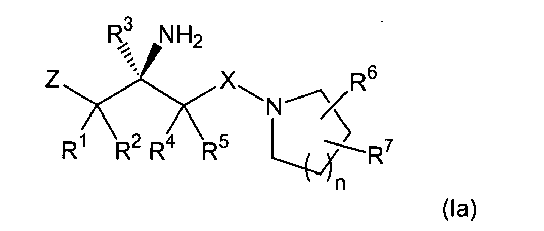 wherein Z, R¹-R⁷, X and n have the meaning as indicated above.

Preferred compounds of formula (I) or (Ia) are those compounds in which one or more of the residues contained therein have the meanings given below, with all combinations of preferred substituent definitions being a subject of the present invention. With respect to all preferred compounds of the formulas (I) or (Ia) the present invention also includes all tautomeric and stereoisomeric forms and mixtures thereof in all ratios, and their pharmaceutically acceptable salts.

In preferred embodiments of the present invention, the substituents Z, R¹-R⁷, X and n of the formula (I) or (Ia) independently from each other have the following meaning. Hence, one or more of the substituents Z, R¹-R⁷, X and n can have the preferred or more preferred meanings given below.

Z is as defined above. Preferably, Z is phenyl or heterocycle. When Z is a heterocycle, it is preferably an aromatic heterocycle.

Preferably, Z is optionally substituted with 1, 2 or 3, in one embodiment 1 or 2, R⁸, which are the same or different. Preferably, they are the same.

R⁸ is as defined above. Preferably, R⁸ is selected from the group consisting of Cl; F; CN; CH₃; and OCH₃, more preferably Cl, F or CN, most preferably F.

In a more preferred embodiment Z is 2-Fluoro-phenyl. In another preferred embodiment Z is 2,4,5-Trifluoro-phenyl.

R¹, R⁴ are independently as defined above. Preferably, R¹, R⁴ are independently selected from the group consisting of H; F; OH; CH₃; and OCH₃, more preferably H or F.

R², R⁵ are independently as defined above. Preferably, R², R⁵ are independently selected from the group consisting of H; F; and CH₃, more preferably H or F.

R¹, R², R⁴, R⁵ are more preferred H.

R³ is as defined above. Preferably, R³ is H.

X is as defined above. Preferably, X is C(O). In another embodiment, X is preferably S(O)₂.

n is as defined above.

In one embodiment, n is 0. In this case, R⁶ is preferably bound in the 2-position. Alternatively, R⁶ is preferably bound in the 3-position.

In another embodiment, n is 1. In this case, R⁶ is preferably bound in the 2-position.

n can also be 2. In this case, R⁶ is preferably bound in the 4-position. Alternatively, R⁶ is preferably bound in the 3-position.
A) When n is 2 and R⁶ is bound in the 4-position, R⁶ is as defined above. Preferably R⁶ is selected from the group consisting of (1); (2) and/or (3), most preferably each of (1); (2) and (3)
   (1) halogen; CN; OH; NH₂; COOH; C(O)NH₂; C₁₋₆ alkyl; O-C₁₋₆ alkyl; N(R^{32a})-C₁₋₆ alkyl; C(O)N(R^{32a})-C₁₋₆ alkyl; N(R^{32a})-C(O)-T³; S(O)N(R^{32a})-C₁₋₆ alkyl; N(R^{32a})S(O)₂T³; N(R^{32a})T³; S(O)₂-T³; S(O)₂-C₁₋₆ alkyl; N(R^{32a})S(O)₂-C₁₋₆ alkyl; C₁₋₆ alkyl-T³; OT³; wherein each C₁₋₆ alkyl is optionally substituted with 1, 2 or 3 OH or halogen independently selected from the group consisting of F; and Cl;
      preferably halogen; CN; OH; C₁₋₄ alkyl; C(O)N(R^{32a})-C₁₋₄ alkyl; N(R^{32a})S(O)₂T³; N(R^{32a})T³; S(O)₂-T³; C₁₋₄ alkyl-T³; OT³; wherein each C₁₋₄ alkyl is optionally substituted with 1, 2 or 3 F or 1 OH;
      more preferably F; Cl; OH; C₁₋₂ alkyl; C(O)NH-C₁₋₂ alkyl; N(cyclopropyl)S(O)₂₋phenyl; NH-phenyl; C₁₋₂ alkyl-phenyl; C₁₋₂ alkyl-phenyl; O-phenyl; wherein each phenyl is optionally substituted with halogen; CN; OH; C₁₋₆ alkyl; O-C₁₋₆ alkyl; S(O)₂-C₁₋₆ alkyl; NHS(O)₂-C₁₋₆ alkyl; wherein each C₁₋₆ alkyl is optionally substituted with one more halogen selected from the group consisting of F; preferably each phenyl is optionally substituted with halogen; C₁₋₂ alkyl; S(O)₂₋C₁₋₂ alkyl; most preferably each phenyl is optionally substituted with F; CF₃ and S(O)₂Me; and wherein each C₁₋₂ alkyl is optionally substituted with 1, 2 or 3 F or 1 OH;
      most preferably F; Cl; OH; C₁₋₂ alkyl; C(O)NH-C₁₋₂ alkyl; N(cyclopropyl)S(O)₂₋phenyl; N(cyclopropyl)S(O)₂-phenyl substituted with 1, 2 or 3, more preferably 1, halogen; NH-phenyl substituted with 1 R⁵¹, more preferably 1 S(O)₂ C₁₋₂ alkyl; S(O)₂-phenyl optionally substituted with C₁₋₂ alkyl; C₁₋₂ alkyl-phenyl optionally substituted with 1 R⁵¹, more preferably 1 methyl; C₁₋₂ alkyl-phenyl; O-phenyl; wherein each C₁₋₂ alkyl is optionally substituted with 1, 2 or 3 F or 1 OH;
   (2) phenyl; naphthyl; and indenyl; which are each optionally substituted with one or more R³⁸; wherein R³⁸ is independently selected from the group consisting of halogen; CN; COOH; OH; C(O)NH₂; S(O)₂NH₂; C₁₋₆ alkyl; O-C₁₋₆ alkyl; S-C₁₋₆ alkyl; COO-C₁₋₆ alkyl; OC(O)- C₁₋₆ alkyl; C(O)N(R⁴⁴)-C₁₋₆ alkyl; S(O)₂N(R⁴⁴)-C₁₋₆ alkyl; S(O)₂-C₁₋₆ alkyl; and N(R⁴⁴)S(O)₂-C₁₋₆ alkyl; wherein each C₁₋₆ alkyl is optionally substituted with one or more R⁴⁵, wherein R⁴⁵ is independently selected from the group consisting of F or OH;
      more preferably phenyl which is optionally substituted with one or more R³⁸; wherein R³⁸ is independently selected from the group consisting of halogen; CN; COOH; OH; C₁₋₄ alkyl; O-C₁₋₄ alkyl; wherein each C₁₋₄ alkyl is optionally substituted with 1, 2 or 3 R⁴⁵, wherein R⁴⁵ is independently selected from the group consisting of F or OH;
      most preferably phenyl which is optionally substituted with one or more R³⁸; wherein R³⁸ is independently selected from the group consisting of Cl; F; OH; C₁₋₂ alkyl; O-C₁₋₂ alkyl; wherein each C₁₋₂ alkyl is optionally substituted with 1, 2 or 3 R⁴⁵, wherein R⁴⁵ is independently selected from the group consisting of F or OH;
   (3) heterocycle; and heterobicycle; which are each optionally substituted with one or more R⁴¹, wherein R⁴¹ is independently selected from the group consisting of halogen; CN; R⁴²; OH; oxo (=O), where the ring is at least partially saturated; NH₂; COOH; C(O)NH₂; S(O)₂NH₂; N(R⁴³)T³; and T³; more preferably (3.1) a heterocycle selected from a 6-membered N-containing ring which may be saturated or aromatic, preferably containing 1 or 2 N atoms, or a 5-membered N-containing aromatic ring, preferably containing 1 or 2 N atoms, more preferably piperidyl, morpholinyl; pyridyl; pyrazinyl; pyrimidyl, and oxadiazole preferably selected from 1,3,4-oxadiazole and 1,2,4-oxadiazole; or (3.2) a heterobicycle selected from fused N-containing rings, preferably a benzyl ring fused to a N-containing 5-membered ring which is preferably saturated; more preferably a heterobicycle selected from 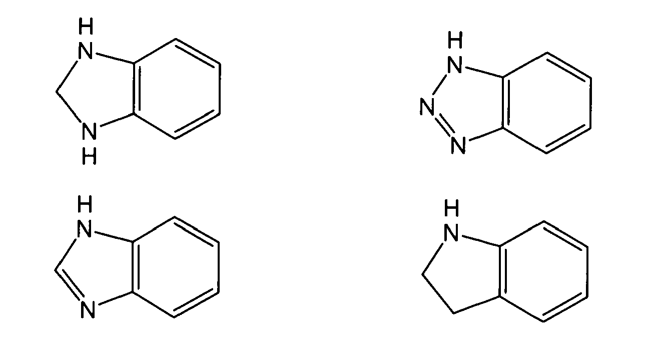 whereby both the heterocycle and the heterobicycle are each optionally substituted with one or more, more preferably 1 or 2, most preferably 1, R⁴¹, wherein R⁴¹ is independently selected from the group consisting of halogen; CN; R⁴²; OH; oxo (=O), where the ring is at least partially saturated; NH₂; COOH; C(O)NH₂; S(O)₂NH₂; N(R⁴³)T³; and T³; more preferably R⁴¹ is independently selected from the group consisting of
      halogen; CN; OH; oxo (=O), where the ring is at least partially saturated; NH₂; COOH; C(O)NH₂; C₁₋₄ alkyl; O-C₁₋₄ alkyl; phenyl optionally substituted with halogen; CN; OH; C₁₋₆ alkyl; O-C₁₋₆ alkyl; S(O)₂₋C₁₋₆ alkyl; NHS(O)₂-C₁₋₆ alkyl; wherein each C₁₋₆ alkyl is optionally substituted with one more halogen selected from the group consisting of F; preferably halogen; C₁₋₂ alkyl; S(O)₂-C₁₋₂ alkyl; most preferably F; CF₃ and S(O)₂Me; and
      heterocycle optionally substituted with halogen; CN; OH; C₁₋₆ alkyl; O-C₁₋₆ alkyl; S(O)₂-C₁₋₆ alkyl; NHS(O)₂-C₁₋₆ alkyl; preferably a 5- or 6-membered, more preferably 6-membered, N-containing heterocycle which is preferably aromatic, more preferably pyridyl and pyrazinyl;
      most preferably R⁴¹ is independently selected from the group consisting of halogen, e.g. F or Cl; oxo (=O), where the ring is at least partially saturated; phenyl as defined above; and heterocycle as defined above.
B) When n is 0, R⁶ is as defined above. Preferably R⁶ is selected from the group consisting of (1); (2) and/or (3), most preferably (1) and (3)
   (1) halogen; CN; OH; NH₂; COOH; C(O)NH₂; S(O)₂NH₂; S(O)NH₂; C₁₋₆ alkyl; O-C₁₋₆ alkyl; N(R^{32a})-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆ alkyl-T³; C(O)N(R^{32a})-T³; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; C₁₋₆ alkyl-T³; OT³; wherein each C₁₋₆ alkyl is optionally substituted with one or more OH or halogen independently selected from the group consisting of F; and Cl;
      preferably halogen; CN; OH; NH₂; COOH; C(O)NH₂; C₁₋₆ alkyl; O-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆ alkyl-T³; C(O)N(R^{32a})-T³; N(R^{32a})T³; S(O)₂-T³; S(O)₂-C₁₋₆ alkyl; N(R^{32a})S(O)₂-C₁₋₆ alkyl; N(R^{32a})S(O)-C₁₋₆ alkyl; C₁₋₆ alkyl-T³; OT³; wherein each C₁₋₆ alkyl is optionally substituted with one or more OH or halogen independently selected from the group consisting of F; and Cl;
      more preferably halogen; OH; C₁₋₄ alkyl; O-C₁₋₄ alkyl; C(O)N(R^{32a})-C₁₋₄ alkyl-T³; C(O)N(R^{32a})-T³; wherein each C₁₋₆ alkyl is optionally substituted with one OH or 1, 2 or 3 halogen independently selected from the group consisting of F; and Cl;
      most preferably C(O)NH-C₁₋₂ alkyl-T³; or C(O)NH-T³; wherein T³ is preferably phenyl or a 5- or 6-membered N-containing heterocycle which is preferably aromatic, more preferably phenyl or pyridyl each independently optionally substituted with halogen; CN; OH; C₁₋₆ alkyl; O-C₁₋₆ alkyl; S(O)₂-C₁₋₆ alkyl; NHS(O)₂-C₁₋₆ alkyl;
   (2) phenyl; naphthyl; and indenyl; which are each substituted with one or more R³⁸; wherein R³⁸ is independently selected from the group consisting of R³⁹; COOT³; OT³; ST³; C(O)N(R⁴⁰)T³; S(O)₂N(R⁴⁰)T³; S(O)N(R⁴⁰)T³ and T³; preferably phenyl which is optionally substituted with one or more R³⁸; wherein R³⁸ is independently selected from the group consisting of halogen; CN; COOH; OH; C₁₋₄ alkyl; O-C₁₋₄ alkyl; wherein each C₁₋₄ alkyl is optionally substituted with 1, 2 or 3 R⁴⁵, wherein R⁴⁵ is independently selected from the group consisting of F or OH; most preferably phenyl which is optionally substituted with one or more R³⁸; wherein R³⁸ is independently selected from the group consisting of Cl; F; OH; C₁₋₂ alkyl; O-C₁₋₂ alkyl;
   (3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of R⁴²; COOT³; OT³; C(O)N(R⁴³)T³; S(O)₂N(R⁴³)T³; S(O)N(R⁴³)T³; N(R⁴³)T³; and T³; preferably heterocycle; and heterobicycle, more preferably heterocycle which is preferably a 5- or 6-membered, more preferably 6-membered, N-containing ring which is preferably saturated, yet more preferably piperidyl or piperazinyl; which are each optionally substituted with one or more R⁴¹, wherein R⁴¹ is independently selected from the group consisting of OT³; N(R⁴³)T³; and T³; more preferably T³; yet more preferably phenyl or a 5- or 6-membered, more preferably 6-membered, N-containing heterocycle which is preferably aromatic, most preferably pyridyl and pyrazinyl.
   In the case of (B) R³⁹ is preferably selected from the group consisting of S(O)-C₁₋₆ alkyl and S(O)₂-C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³; and
   R⁴² is preferably N(R⁴⁸)-C(O)-C₁₋₆ alkyl wherein C₁₋₆ alkyl is optionally substituted with one or more R⁴⁵, wherein R⁴⁵ is independently selected from the group consisting of F; COOR⁴⁹; C(O)N(R⁴⁹R⁵⁰); S(O)₂N(R⁴⁹R⁵⁰); S(O)N(R⁴⁹R⁵⁰); OR⁴⁹; N(R⁴⁹R⁵⁰); T³; O-T³; and N(R⁴⁹)-T³.
C) When n is 2 and R⁶ is bound in the 3-position, R⁶ is as defined above. Preferably R⁶ is selected from the group consisting of (1); (2) and/or (3), most preferably (1)
   (1) halogen; CN; OH; NH₂; COOH; C(O)NH₂; S(O)₂NH₂; S(O)NH₂; C₁₋₆ alkyl; O-C₁₋₆ alkyl; N(R^{32a})-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆ alkyl-T³; C(O)N(R^{32a})-T³; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; C₁₋₆ alkyl-T³; OT³; wherein each C₁₋₆ alkyl is optionally substituted with one or more OH or halogen independently selected from the group consisting of F; and Cl;
      preferably halogen; CN; OH; NH₂; COOH; C(O)NH₂; S(O)₂NH₂; C₁₋₆ alkyl; O-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆ alkyl-T³; C(O)N(R^{32a})-T³; C₁₋₆ alkyl-T³; O-T³; wherein each C₁₋₆ alkyl is optionally substituted with one or more OH or halogen independently selected from the group consisting of F; and Cl;
      more preferably halogen; CN; OH; NH₂; COOH; C(O)NH₂; C₁₋₄ alkyl; O-C₁₋₄ alkyl; COO-C₁₋₆ alkyl; C(O)N(R^{32a})-C₁₋₄ alkyl; C(O)N(R^{32a})-C₁₋₄ alkyl-T³; C₁₋₄ alkyl-T³; O-T³;
      yet more preferably F; Cl; C₁₋₂ alkyl; C(O)NHC₁₋₄ alkyl; C(O)N(C₁₋₄ alkyl)C₁₋₄ alkyl;
      C₁₋₄ alkyl-phenyl optionally substituted with halogen; CN; OH; C₁₋₆ alkyl; O-C₁₋₆ alkyl; S(O)₂-C₁₋₄ alkyl; NHS(O)₂-C₁₋₄ alkyl; wherein each C₁₋₄ alkyl is optionally substituted with 1 OH or 1, 2 or 3 halogen independently selected from the group consisting of F; and Cl;
      O-phenyl optionally substituted with halogen; CN; OH; C₁₋₆ alkyl; O-C₁₋₆ alkyl; S(O)₂-C₁₋₄ alkyl; NHS(O)₂-C₁₋₄ alkyl; wherein each C₁₋₄ alkyl is optionally substituted with 1 OH or 1, 2 or 3 halogen independently selected from the group consisting of F; and Cl; wherein each alkyl is optionally substituted with 3 F;
      most preferably F; CF₃; C(O)N(ethyl)₂; CH₂-2-Cl-phenyl; O-phenyl;
   (2) phenyl; naphthyl; and indenyl; which are each substituted with one or more R³⁸; wherein R³⁸ is independently selected from the group consisting of R³⁹; COOT³; OT³; ST³; C(O)N(R⁴⁰)T³; S(O)₂N(R⁴⁰)T³; S(O)N(R⁴⁰)T³ and T³; preferably phenyl which is optionally substituted with one or more R³⁸; wherein R³⁸ is independently selected from the group consisting of halogen; CN; COOH; OH; C₁₋₄ alkyl; O-C₁₋₄ alkyl; wherein each C₁₋₄ alkyl is optionally substituted with 1, 2 or 3 R⁴⁵, wherein R⁴⁵ is independently selected from the group consisting of F or OH; most preferably phenyl which is optionally substituted with one or more R³⁸; wherein R³⁸ is independently selected from the group consisting of Cl; F; OH; C₁₋₂ alkyl; O-C₁₋₂ alkyl;
   (3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of R⁴²; COOT³; OT³; C(O)N(R⁴³)T³; S(O)₂N(R⁴³)T³; S(O)N(R⁴³)T³; N(R⁴³)T³; and T³; more preferably heterocycle; and heterobicycle; which are each optionally substituted with one or more R⁴¹, wherein R⁴¹ is independently selected from the group consisting T³; more preferably (3.1) a heterocycle selected from a 6-membered N-containing ring which may be saturated or aromatic, preferably containing 1 or 2 N atoms, or a 5-membered N-containing aromatic ring, preferably containing 1 or 2 N atoms, more preferably piperidyl, pyrimidyl, and oxadiazole preferably selected from 1,3,4-oxadiazole and 1,2,4-oxadiazole; or (3.2) a heterobicycle selected from fused N-containing rings, preferably a benzyl ring fused to a N-containing 5-membered ring which is preferably saturated; whereby both the heterocycle and the heterobicycle are each optionally substituted with one or more, more preferably 1 or 2, most preferably 1,
      phenyl optionally substituted with halogen; CN; OH; C₁₋₆ alkyl; O-C₁₋₆ alkyl; S(O)₂-C₁₋₆ alkyl; NHS(O)₂-C₁₋₆ alkyl; wherein each C₁₋₆ alkyl is optionally substituted with one more halogen selected from the group consisting of F; preferably halogen; C₁₋₂ alkyl; S(O)₂-C₁₋₂ alkyl; most preferably F; CF₃ and
      S(O)₂Me; and
      heterocycle optionally substituted with halogen; CN; OH; C₁₋₆ alkyl; O-C₁₋₆ alkyl; S(O)₂-C₁₋₆ alkyl; NHS(O)₂-C₁₋₆ alkyl, preferably a 5- or 6-membered, more preferably 6-membered, N-containing heterocycle which is preferably aromatic, more preferably pyridyl and pyrazinyl.
   In the case of (C) R³⁹ is preferably selected from the group consisting of S(O)-C₁₋₆ alkyl and S(O)₂-C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³; and
   R⁴² is preferably N(R⁴⁸)-C(O)-C₁₋₆ alkyl wherein C₁₋₆ alkyl is optionally substituted with one or more R⁴⁵, wherein R⁴⁵ is independently selected from the group consisting of ,F; COOR⁴⁹; C(O)N(R⁴⁹R⁵⁰); S(O)₂N(R⁴⁹R⁵⁰); S(O)N(R⁴⁹R⁵⁰); OR⁴⁹; N(R⁴⁹R⁵⁰); T³; O-T³; and N(R⁴⁹)-T³.
D) When n is 2 and R⁶ is bound in the 2-position, R⁶ is as defined above. Preferably R⁶ is selected from the group consisting of (1); (2) and/or (3), most preferably (1)
   R⁶ is selected from the group consisting of
   (1) halogen; OH; NH₂; COOH; C(O)NH₂; S(O)₂NH₂; S(O)NH₂; O-C₁₋₆ alkyl; N(R^{32a})-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆ alkyl-T³; C(O)N(R^{32a})-T³; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; OT³; wherein each C₁₋₆ alkyl is optionally substituted with one or more OH or halogen independently selected from the group consisting of F; and Cl; preferably OH; NH₂; S(O)₂NH₂; S(O)NH₂; O-C₁₋₆ alkyl; N(R^{32a})-C₁₋₆ alkyl; OC(O)-C₁₋₆ alkyl; C(O)N(R^{32a})-C₁₋₆ alkyl-T³; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; OT³; wherein each C₁₋₆ alkyl is optionally substituted with one or more OH or halogen independently selected from the group consisting of F; and Cl; more preferably OH; NH₂; O-C₁₋₄ alkyl; C(O)N(R^{32a})-C₁₋₄ alkyl-T³; wherein each C₁₋₄ alkyl is optionally substituted with 1 OH or 1, 2 or 3 halogen independently selected from the group consisting of F; and Cl;
   (2) phenyl; naphthyl; and indenyl; which are each substituted with one or more R³⁸; wherein R³⁸ is independently selected from the group consisting of R³⁹; COOT³; OT³; ST³; C(O)N(R⁴⁰)T³; S(O)₂N(R⁴⁰)T³; S(O)N(R⁴⁰)T³ and T³; preferably phenyl which is optionally substituted with one or more R³⁸; wherein R³⁸ is independently selected from the group consisting of halogen; CN; COOH; OH; C₁₋₄ alkyl; O-C₁₋₄ alkyl; wherein each C₁₋₄ alkyl is optionally substituted with 1, 2 or 3 R⁴⁵, wherein R⁴⁵ is independently selected from the group consisting of F or OH; most preferably phenyl which is optionally substituted with one or more R³⁸; wherein R³⁸ is independently selected from the group consisting of Cl; F; OH; C₁₋₂ alkyl; O-C₁₋₂ alkyl;
   (3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of R⁴²; COOT³; OT³; C(O)N(R⁴³)T³; S(O)₂N(R⁴³)T³; S(O)N(R⁴³)T³; N(R⁴³)T³; and T³; more preferably heterocycle; and heterobicycle; which are each optionally substituted with one or more R⁴¹, wherein R⁴¹ is independently selected from the group consisting T³; more preferably (3.1) a heterocycle selected from a 6-membered N-containing ring which may be saturated or aromatic, preferably containing 1 or 2 N atoms, or a 5-membered N-containing aromatic ring, preferably containing 1 or 2 N atoms, more preferably piperidyl, pyrimidyl, and oxadiazole preferably selected from 1,3,4-oxadiazole and 1,2,4-oxadiazole; or (3.2) a heterobicycle selected from fused N-containing rings, preferably a benzyl ring fused to a N-containing 5-membered ring which is preferably saturated; whereby both the heterocycle and the heterobicycle are each optionally substituted with one or more, more preferably 1 or 2, most preferably 1,
      phenyl optionally substituted with halogen; CN; OH; C₁₋₆ alkyl; O-C₁₋₆ alkyl; S(O)₂-C₁₋₆ alkyl; NHS(O)₂-C₁₋₆ alkyl; wherein each C₁₋₆ alkyl is optionally substituted with one more halogen selected from the group consisting of F; preferably halogen; C₁₋₂ alkyl; S(O)₂-C₁₋₂ alkyl; most preferably F; CF₃ and S(O)₂Me; and
      heterocycle optionally substituted with halogen; CN; OH; C₁₋₆ alkyl; O-C₁₋₆ alkyl; S(O)₂-C₁₋₆ alkyl; NHS(O)₂-C₁₋₆ alkyl, preferably a 5- or 6-membered, more preferably 6-membered, N-containing heterocycle which is preferably aromatic, more preferably pyridyl and pyrazinyl.
   In the case of (D) R³⁹ is preferably selected from the group consisting of S(O)-C₁₋₆ alkyl and S(O)₂-C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³; and
   R⁴² is preferably N(R⁴⁸)-C(O)-C₁₋₆ alkyl wherein C₁₋₆ alkyl is optionally substituted with one or more R⁴⁵, wherein R⁴⁵ is independently selected from the group consisting of F; COOR⁴⁹; C(O)N(R⁴⁹R⁵⁰); S(O)₂N(R⁴⁹R⁵⁰); S(O)N(R⁴⁹R⁵⁰); OR⁴⁹; N(R⁴⁹R⁵⁰); T³; O-T³; and N(R⁴⁹)-T³.
E) When n is 1, R⁶ is as defined above. Preferably R⁶ is selected from the group consisting of (1); (2) and/or (3), most preferably (1)
   (1) OH; NH₂; COOH; C(O)NH₂; S(O)₂NH₂; S(O)NH₂; O-C₁₋₆ alkyl; N(R^{32a})-C₁₋₆ alkyl; COO-C₁₋₆ alkyl; OC(O)-C₁₋₆ alkyl; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl; ₁₋₆ alkyl-T³; OT³; wherein each C₁₋₆ alkyl is optionally substituted with one or more OH or halogen independently selected from the group consisting of F; and Cl;
      with the proviso that when X is C(O), R⁶ is not (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂₋T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl;
      preferably OH; NH₂; COOH; S(O)₂NH₂; S(O)NH₂; O-C₁₋₆ alkyl; N(R^{32a})-C₁₋₆ alkyl; COO-C₁₋₆ alkyl; OC(O)-C₁₋₆ alkyl; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})-T³; S(O)₂-T³; S(O)-C₁₋₆ alkyl; N(R^{32a})S(O)₂-C₁₋₆ alkyl; N(R^{32a})S(O)-C₁₋₆ alkyl; (CH₂)NH₂; (CH₂)N(R^{32a})-C₁₋₆ alkyl; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)N(R^{32a})S(O)₂T³; (CH₂)N(R^{32a})T³; S(O)₂-T³; (CH₂)N(R^{32a})S(O)₂-C₁₋₆ alkyl; (CH₂)N(R^{32a})S(O)-C₁₋₆ alkyl; C₁₋₆ alkyl-T³; OT³; wherein each C₁₋₆ alkyl is optionally substituted with one or more OH or halogen independently selected from the group consisting of F; and Cl;
      more preferably NH₂; N(R^{32a})-C₁₋₆ alkyl; N(R^{32a})-C(O)-C₁₋₆ alkyl; N(R^{32a})-C(O)-T³; S(O)₂N(R^{32a})-C₁₋₆ alkyl; N(R^{32a})S(O)₂T³; N(R^{32a})T³; N(R^{32a})S(O)₂-C₁₋₆ alkyl; (CH₂)N(R^{32a})-C₁₋₆ alkyl; (CH₂)N(R^{32a})-C(O)-C₁₋₆ alkyl; (CH₂)N(R^{32a})-C(O)-T³; (CH₂)N(R^{32a})S(O)₂T³; (CH₂)N(R^{32a})T³; S(O)₂-T³; (CH₂)N(R^{32a})S(O)₂-C₁₋₆ alkyl; C₁₋₆ alkyl-T³; wherein each C₁₋₆ alkyl is optionally substituted with one or more OH or halogen independently selected from the group consisting of F; and Cl; yet more preferably N(R^{32a})-C(O)-T³; (CH₂)N(R^{32a})S(O)₂T³; N(R^{32a})S(O)₂T³; C₁₋₄ alkyl-T³; wherein each C₁₋₄ alkyl is optionally substituted with one OH or 1, 2 or 3 halogen independently selected from the group consisting of F; and Cl; still more preferably
      (CH₂)NH-S(O)₂-phenyl optionally substituted with halogen; CN; OH; C₁₋₆ alkyl; O-C₁₋₆ alkyl; S(O)₂-C₁₋₆ alkyl; NHS(O)₂-C₁₋₆ alkyl; wherein each C₁₋₆ alkyl is optionally substituted with one more halogen selected from the group consisting of F; preferably halogen; C₁₋₂ alkyl; S(O)₂-C₁₋₂ alkyl;
      NH-C(O)-phenyl optionally substituted with halogen; CN; OH; C₁₋₆ alkyl; O-C₁₋₆ alkyl; S(O)₂-C₁₋₆ alkyl; NHS(O)₂-C₁₋₆ alkyl; wherein each C₁₋₆ alkyl is optionally substituted with one more halogen selected from the group consisting of F; preferably halogen; C₁₋₂ alkyl; S(O)₂-C₁₋₂ alkyl;
      NH-C(O)-C₃₋₇ cycloalkyl;
      CH₂-phenyl optionally substituted with halogen; CN; OH; C₁₋₆ alkyl; O-C₁₋₆ alkyl; S(O)₂-C₁₋₆ alkyl; NHS(O)₂-C₁₋₆ alkyl; wherein each C₁₋₆ alkyl is optionally substituted with one more halogen selected from the group consisting of F; preferably halogen; C₁₋₂ alkyl; S(O)₂-C₁₋₂ alkyl; and
      CH₂-heterocycle whereby the heterocycle is optionally substituted with halogen; CN; OH; C₁₋₆ alkyl; O-C₁₋₆ alkyl; S(O)₂-C₁₋₆ alkyl; NHS(O)₂-C₁₋₆ alkyl, preferably a 5- or 6-membered, more preferably a 6-membered, N-containing heterocycle which is preferably aromatic, more preferably CH₂-pyridyl and CH₂-pyrazinyl;
      most preferably (CH₂)NH-S(O)₂-phenyl optionally substituted with F or Cl; NH-C(O)-phenyl optionally substituted with F or Cl; NH-C(O)-C₃₋₇ cyclopropyl; CH₂-pyridyl; CH₂-phenyl optionally substituted with F or Cl.
   (2) phenyl; naphthyl; and indenyl; which are each substituted with one or more R³⁸; wherein R³⁸ is independently selected from the group consisting of R³⁹; COOT³; OT³; ST³; C(O)N(R⁴⁰)T³; S(O)₂N(R⁴⁰)T³; S(O)N(R⁴⁰)T³ and T³; preferably phenyl which is optionally substituted with one or more R³⁸; wherein R³⁸ is independently selected from the group consisting of halogen; CN; COOH; OH; C₁₋₄ alkyl; O-C₁₋₄ alkyl; wherein each C₁₋₄ alkyl is optionally substituted with 1, 2 or 3 R⁴⁵, wherein R⁴⁵ is independently selected from the group consisting of F or OH; most preferably phenyl which is optionally substituted with one or more R³⁸; wherein R³⁸ is independently selected from the group consisting of Cl; F; OH; C₁₋₂ alkyl; O-C₁₋₂ alkyl;
   (3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of R⁴²; COOT³; OT³; C(O)N(R⁴³)T³; S(O)₂N(R⁴³)T³; S(O)N(R⁴³)T³; N(R⁴³)T³; and T³; more preferably heterocycle; and heterobicycle; which are each optionally substituted with one or more R⁴¹, wherein R⁴¹ is independently selected from the group consisting T³; more preferably (3.1) a heterocycle selected from a 6-membered N-containing ring which may be saturated or aromatic, preferably containing 1 or 2 N atoms, or a 5-membered N-containing aromatic ring, preferably containing 1or 2 N atoms, more preferably piperidyl, pyrimidyl, and oxadiazole preferably selected from 1,3,4-oxadiazole and 1,2,4-oxadiazole; or (3.2) a heterobicycle selected from fused N-containing rings, preferably a benzyl ring fused to a N-containing 5-membered ring which is preferably saturated; whereby both the heterocycle and the heterobicycle are each optionally substituted with one or more, more preferably 1 or 2, most preferably 1,
      phenyl optionally substituted with halogen; CN; OH; C₁₋₆ alkyl; O-C₁₋₆ alkyl; S(O)₂-C₁₋₆ alkyl; NHS(O)₂-C₁₋₆ alkyl; wherein each C₁₋₆ alkyl is optionally substituted with one more halogen selected from the group consisting of F; preferably halogen; C₁₋₂ alkyl; S(O)₂-C₁₋₂ alkyl; most preferably F; CF₃ and S(O)₂Me; and
      heterocycle optionally substituted with halogen; CN; OH; C₁₋₆ alkyl; O-C₁₋₆ alkyl; S(O)₂-C₁₋₆ alkyl; NHS(O)₂-C₁₋₆ alkyl, preferably a 5- or 6-membered, more preferably 6-membered, N-containing heterocycle which is preferably aromatic, more preferably pyridyl and pyrazinyl.
   In the case of (E) R³⁹ is preferably selected from the group consisting of S(O)-C₁₋₆ alkyl and S(O)₂-C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³; and
   R⁴² is preferably N(R⁴⁸)-C(O)-C₁₋₆ alkyl wherein C₁₋₆ alkyl is optionally substituted with one or more R⁴⁵, wherein R⁴⁵ is independently selected from the group consisting of F; COOR⁴⁹; C(O)N(R⁴⁹R⁵⁰); S(O)₂N(R⁴⁹R⁵⁰); S(O)N(R⁴⁹R⁵⁰); OR⁴⁹; N(R⁴⁹R⁵⁰); T³; O-T³; and N(R⁴⁹)-T³.
   R^{32a} is as defined above. Preferably R^{32a} is selected from the group consisting of H; C₃₋₇ cycloalkyl and C₁₋₄ alkyl more preferably H; cyclopropyl; methyl and ethyl.
   R³⁹ is as defined above. Preferably R³⁹ is selected from the group consisting of C₁₋₆ alkyl; O-C₁₋₆ alkyl; S-C₁₋₆ alkyl; wherein each C₁₋₆ alkyl is optionally substituted with one or more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³; more preferably R³⁹ is C₁₋₄ alkyl or O-C₁₋₄ alkyl; wherein each C₁₋₄ alkyl is optionally substituted with 1, 2 or 3 F.
   R⁴² is as defined above. Preferably R⁴² is selected from the group consisting of C₁₋₆ alkyl; O-C₁₋₆ alkyl; S-C₁₋₆ alkyl; wherein each C₁₋₆ alkyl is optionally substituted with one or more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³; more preferably R³⁹ is C₁₋₄ alkyl or O-C₁₋₄ alkyl; wherein each C₁₋₄ alkyl is optionally substituted with 1, 2 or 3 F.
   R⁴⁰, R⁴³, R⁴⁴, R⁴⁶, R⁴⁷, R⁴⁸, R⁴⁹, R⁵⁰ are as defined above. Preferably they are independently selected from the group consisting of H; and C₁₋₂ alkyl; most preferably H.
   T³ is selected from the group consisting of T⁴; and T⁵.
   T⁴ is as defined above. Preferably T⁴ is selected from the group consisting of phenyl and naphthyl; more preferably phenyl; wherein T⁴ is optionally substituted with one or more R⁵¹, wherein R⁵¹ is independently selected from the group consisting of halogen; CN; COOR⁵²; OR⁵²; C(O)N(R⁵²R⁵³); S(O)₂N(R⁵²R⁵³); C₁₋₆ alkyl; O-C₁₋₆ alkyl; S-C₁₋₆ alkyl; COO-C₁₋₆ alkyl; OC(O)-C₁₋₆ alkyl; C(O)N(R⁵²)- C₁₋₆ alkyl; S(O)₂N(R⁵²)-C₁₋₆ alkyl; S(O)N(R⁵²)-C₁₋₆ alkyl; S(O)₂₋C₁₋₆ alkyl; S(O) -C₁₋₆ alkyl; N(R⁵²)S(O)₂-C₁₋₆ alkyl; and N(R⁵²)S(O)-C₁₋₆ alkyl; wherein each C₁₋₆ alkyl is optionally substituted with one more halogen selected from the group consisting of F; and Cl;
   T⁵ is as defined above. Preferably T⁵ is selected from the group consisting of heterocycle and heterobicycle; wherein T⁵ is optionally substituted with one or more R⁵⁴, wherein R⁵⁴ is independently selected from the group consisting of halogen; CN; OR⁵⁵; oxo (=O), where the ring is at least partially saturated; N(R⁵⁵R⁵⁶); COOR⁵⁵; C(O)N(R⁵⁵R⁵⁶); S(O)₂N(R⁵⁵R⁵⁶); S(O)N(R⁵⁵R⁵⁶); C₁₋₆ alkyl; O-C₁₋₆ alkyl; S-C₁₋₆ alkyl; N(R⁵⁵)-C₁₋₆ alkyl; COO-C₁₋₆ alkyl; OC(O)-C₁₋₆ alkyl; C(O)N(R⁵⁵)- C₁₋₆ alkyl; N(R⁵⁵)-C(O)-C₁₋₆ alkyl; S(O)₂N(R⁵⁵)-C₁₋₆ alkyl; S(O)N(R⁵⁵)-C₁₋₆ alkyl; S(O)₂-C₁₋₆ alkyl; S(O)-C₁₋₆ alkyl; N(R⁵⁵)S(O)₂-C₁₋₆ alkyl; and N(R⁵⁵)S(O)-C₁₋₆ alkyl; wherein each C₁₋₆ alkyl is optionally substituted with one more halogen selected from the group consisting of F; and Cl.
   R⁵², R⁵³, R⁵⁵, R⁵⁶, are as defined above. Preferably they are independently selected from the group consisting of H; and C₁₋₂ alkyl; more preferably H.
   R⁷ is as defined above. Preferably R⁷ is selected from the group consisting of hydrogen, halogen; CN; or C₁₋₆ alkyl, more preferably hydrogen, halogen; CN; or C₁₋₂ alkyl, most preferably hydrogen, Cl; F; or CN. In one embodiment R⁷ is H.

Compounds of the formula (I) or (Ia) in which some or all of the above-mentioned groups have the preferred or more preferred meanings are also an object of the present invention.

Preferred examples of the compounds of the present invention include the following:

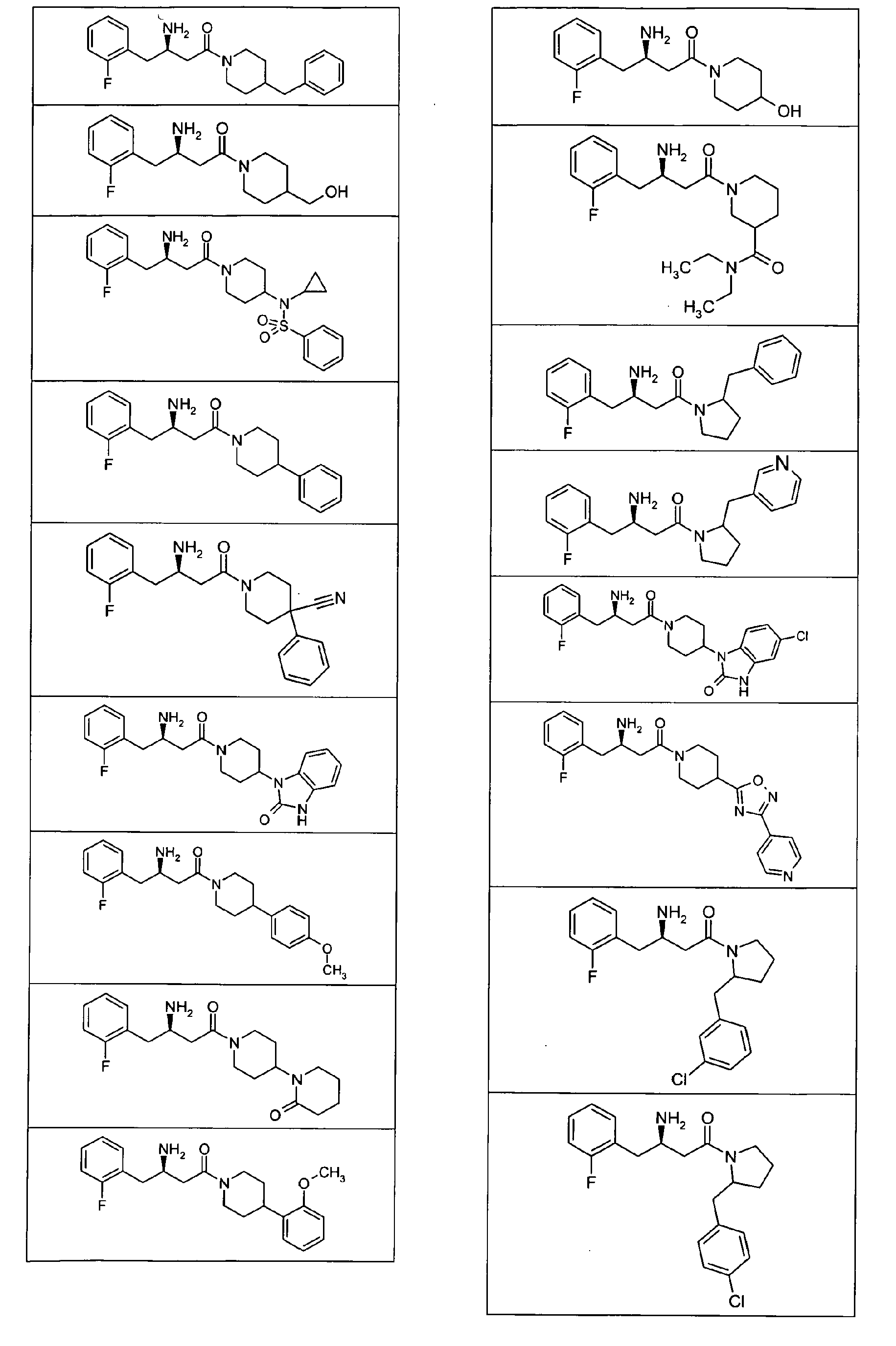

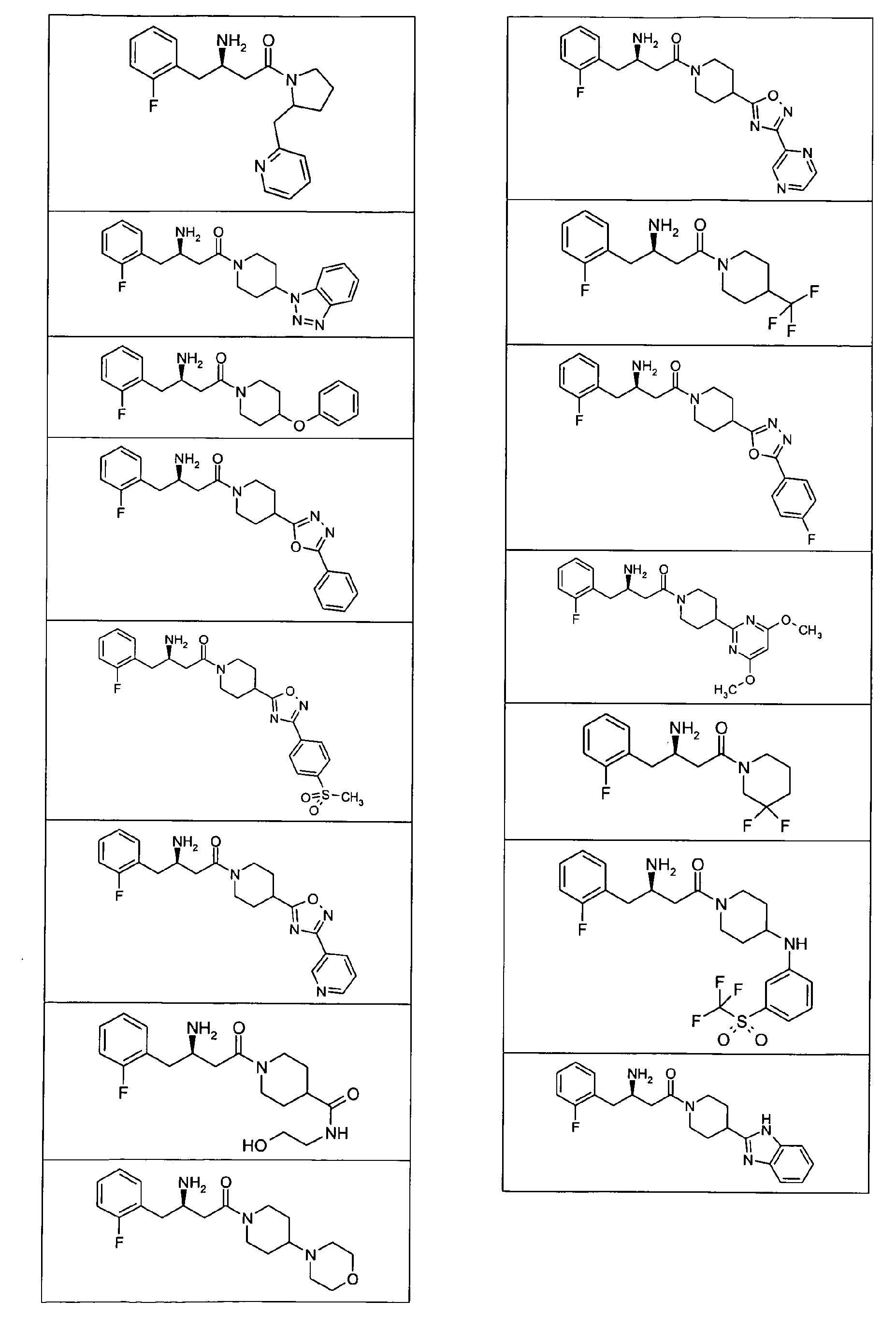

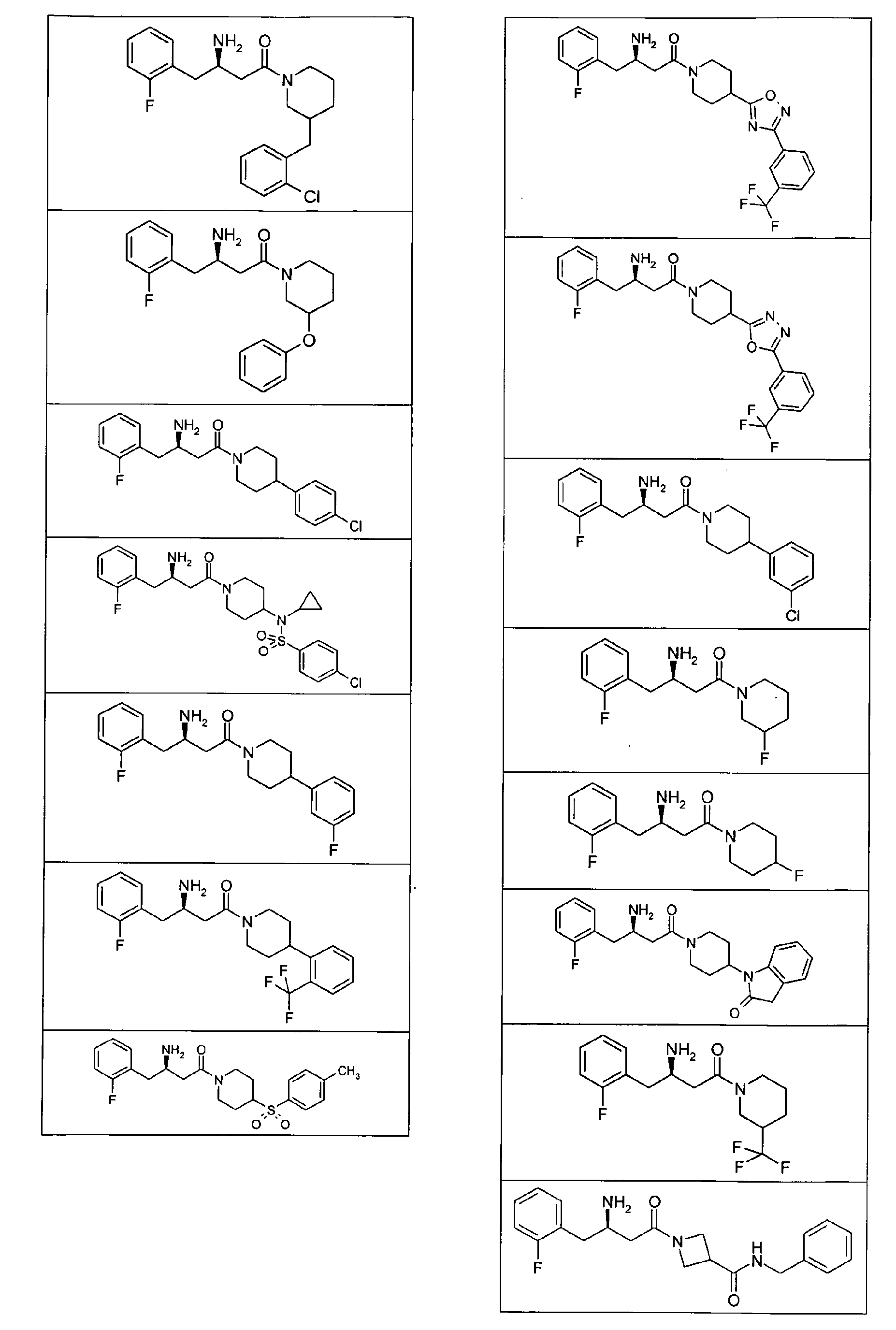

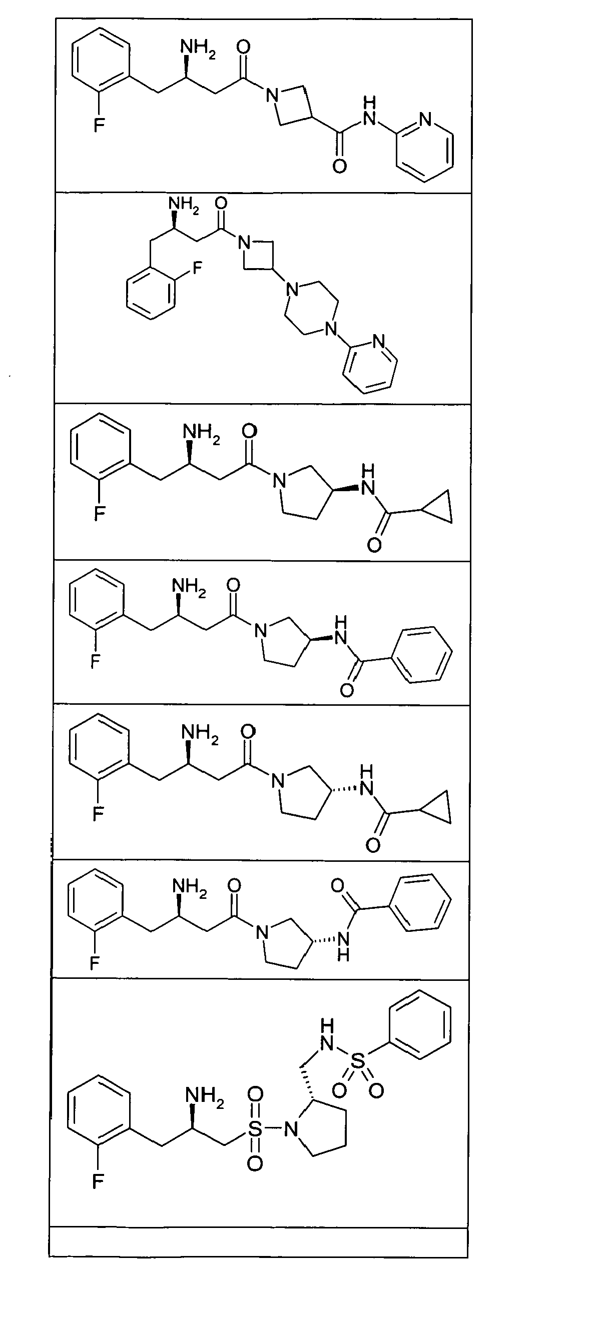

Furthermore, the present invention provides prodrug compounds of the compounds of the invention as described above.

"Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

Metabolites of compounds of formula (I) or (Ia) are also within the scope of the present invention.

Where tautomerism, like e.g. keto-enol tautomerism, of compounds of general formula (I) or (Ia) or their prodrugs may occur, the individual forms, like e.g. the keto and enol form, are claimed separately and together as mixtures in any ratio. Same applies for stereoisomers, like e.g. enantiomers, cis/trans isomers, conformers and the like.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) or (la) may be obtained from stereoselective synthesis using optically pure starting materials.

In case the compounds according to formula (I) or (Ia) contain one or more acidic or basic groups, the invention also comprises their corresponding pharmaceutically or toxicologically acceptable salts, in particular their pharmaceutically utilizable salts. Thus, the compounds of the formula (I) or (Ia) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) or (Ia) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) or (Ia) simultaneously contain acidic and basic groups in the molecule, the invention also includes, in addition to the salt forms mentioned, inner salts or betaines (zwitterions). The respective salts according to the formula (I) or (Ia) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) or (Ia) which, owing to low physiological compatibility, are not directly suitable for use in pharmaceuticals but which can be used, for example, as intermediates for chemical reactions or for the preparation of pharmaceutically acceptable salts.

The present invention provides compounds of general formula (I) or (Ia) or their prodrugs as DPP-IV inhibitors. DPP-IV is a cell surface protein that has been implicated in a wide range of biological functions. It has a broad tissue distribution (intestine, kidney, liver, pancreas, placenta, thymus, spleen, epithelial cells, vascular endothelium, lymphoid and myeloid cells, serum), and distinct tissue and cell-type expression levels. DPP-IV is identical to the T cell activation marker CD26, and it can cleave a number of immunoregulatory, endocrine, and neurological peptides *in vitro.* This has suggested a potential role for this peptidase in a variety of disease processes.

DPP-IV related diseases are described in more detail in WO-A-03/181 under the paragraph "Utilities" which is herewith incorporated by reference.

Accordingly, the present invention provides compounds of formula (I) or (Ia) or their prodrugs or pharmaceutically acceptable salt thereof for use as a medicament.

Furthermore, the present invention provides the use of compounds of formula (I) or (Ia) or their prodrugs or a pharmaceutically acceptable salt thereof for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; anxiety; depression; retinopathy; nephropathy; neuropathy; Syndrome X; ovarian hyperandrogenism (polycystic ovarian syndrome; Type n diabetes; or growth hormone deficiency. Preferred is non-insulin dependent (Type II) diabetes mellitus and obesity.

The present invention provides pharmaceutical compositions comprising a compound of formula (I) or (Ia), or a prodrug compound thereof, or a pharmaceutically acceptable salt thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

A pharmaceutical composition of the present invention may additionally comprise one or more other compounds as active ingredients like one or more additional compounds of formula (I) or (Ia), or a prodrug compound or other DPP-IV inhibitors.

Other active ingredients are disclosed in WO-A-03/181 under the paragraph "Combination Therapy" which is herewith incorporated by reference.

Accordingly, other active ingredients may be insulin sensitizers; PPAR agonists; biguanides; protein tyrosinephosphatase-IB (PTP-1 B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoly dual agonists; inhibitors of cholesterol absorption; acyl CoA : cholesterol acyltransferase inhibitors; anti-oxidants; PPARo agonists; antiobesity compounds; an ileal bile acid transporter inhibitor; or anti-inflammatory agents or pharmaceutically acceptable salts of these active compounds.

The term "pharmaceutically acceptable salts" refers to salts prepared from pharmaceutically acceptable non-toxic bases or acids, including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I) or (Ia) can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) or (Ia) may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) or (Ia) are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing diabetes mellitus and/or hyperglycemia or hypertriglyceridemia or other diseases for which compounds of Formula I are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligrams to about 50 milligrams. In the case of a 70 kg adult human, the total daily dose will generally be from about 7 milligrams to about 350 milligrams. This dosage regimen may be adjusted to provide the optimal therapeutic response.

The compounds of formula (I) of the present invention can be prepared from beta amino acid intermediates such as those of formula (IV) and substituted amine intermediates such as those of formula (III), using standard peptide coupling conditions. The preparation of these intermediates is described in the following schemes.

### Some abbreviations that may appear in this application are as follows.

### ABBREVIATIONS

### Designation

- bs: Broad singlet
- bm: Broad multiplet
- Boc (or BOC): *tert*-Butoxycarbonyl
- CDI: *N*,*N*-Carbonyldiimidazole
- DCE: 1,2-Dichloroethane
- DCM: Dichloromethane
- DIEA: Diisopropylethylamine
- DMF: *N*,*N*-Dimethylformamide
- EDC: 1-Ethyl-3-(3-dimethylaminopropyl)carbodiimide hydrochloride
- Et₃N: Triethylamine
- HATU: *O*-(7-azabenzotriazol-1-yl)-N,N,N',N'-tetramethyluronium hexafluorophosphate
- HBTU: *O*-(benzotrialzol-1-yl)-*N*,*N*,*N*',*N*'-*tetramethylor* hexafluorophosphate
- HCl: Hydrogen chloride
- HOBt: 1-Hydroxybenzotriazole
- HPLC: High pressure liquid chromatography
- M.P.: Melting point
- NMR: Nuclear Magnetic Resonance
- PG: Protecting group
- rt: Retention time
- TFA: Trifluoroacetic acid
- TLC: Thin Layer Chromatography

Available starting materials may be amines having the formula (III).

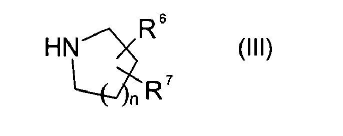

They may be purchased from commercially available sources such as Acros, Astatech, Array, Sigma-Aldrich, Fluka, ABCR or be synthesized by one skilled in the art. Common reactions between compounds containing amino groups and carboxyl, sulphonyl or isocyanate functionalities may be employed for their synthesis with suitable functionalized starting materials. Nucleophilic substitution reactions between compounds containing a suitable leaving group (e.g., halogenide, mesylate, tosylate) and nucleophiles (e.g., amines) may be also employed. The conversion of diverse functional groups (such as esters, alcohols, amides, nitriles, azides) may allow the synthesis of some intermediates or final compounds.

Schemes A through F outline general procedures for the synthesis of some compounds described below. Unless otherwise indicated in the schemes, the variables have the same meaning as described above.

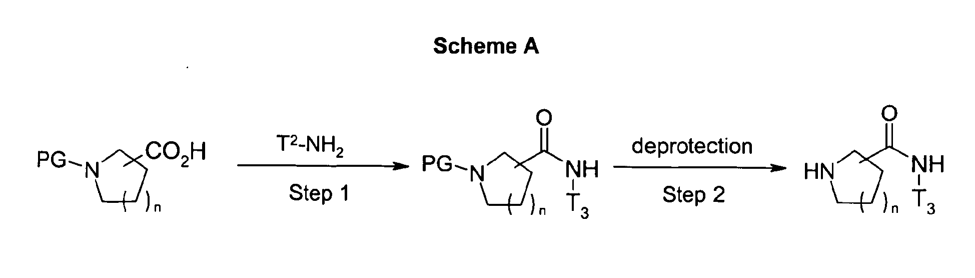

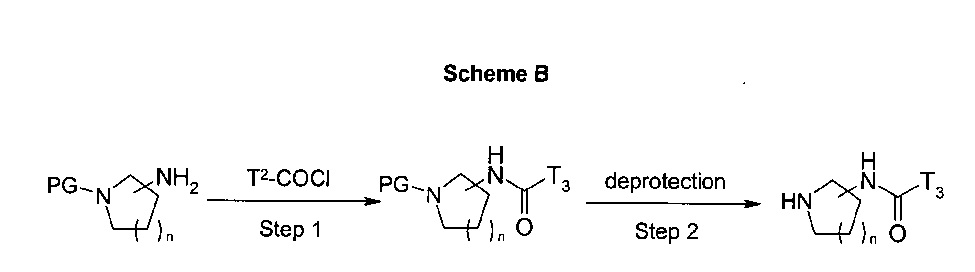

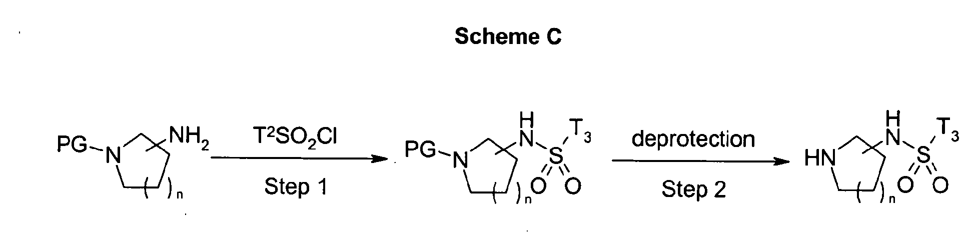

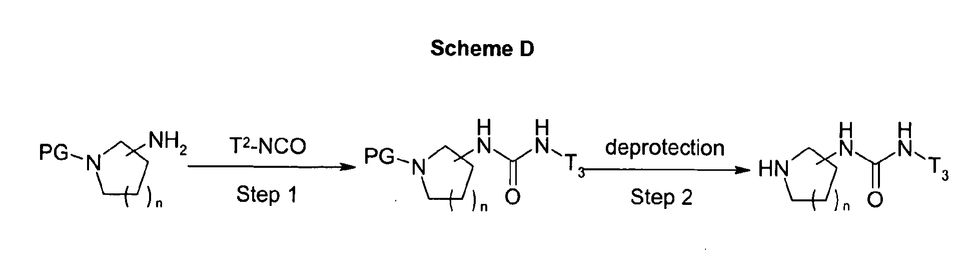

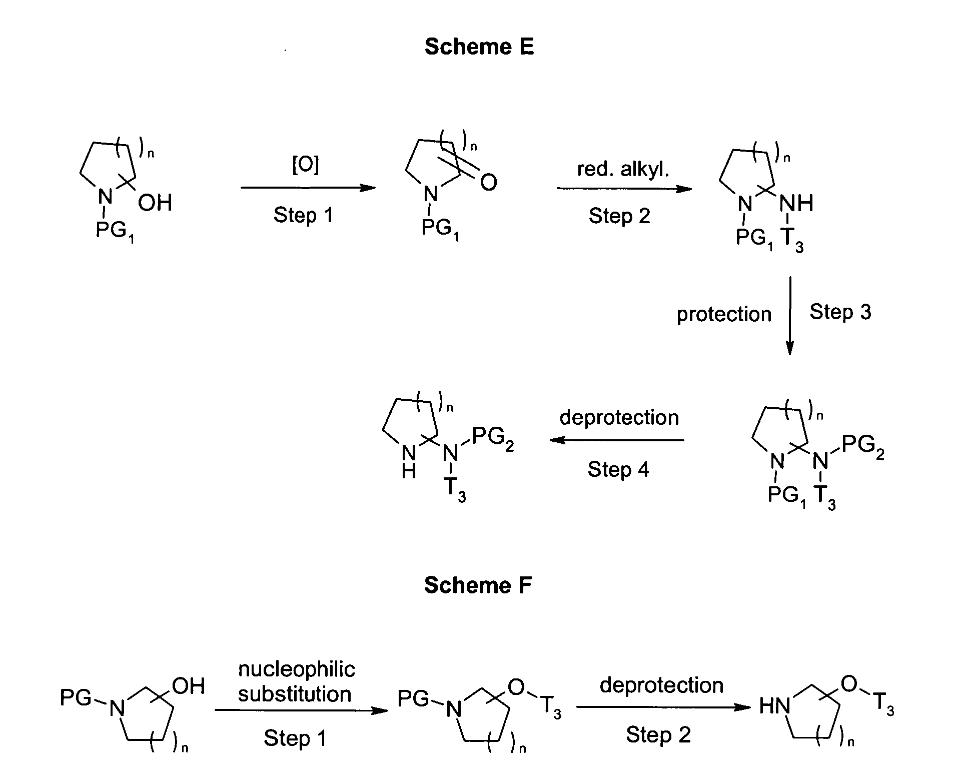

Further substituents contained within compounds of formula (III), for example such as heterocycles or heterobicycles, could be prepared as outlined in the previous application EPO 04/013512.1.

Enantiomerically pure beta amino acids having the formula (IV) 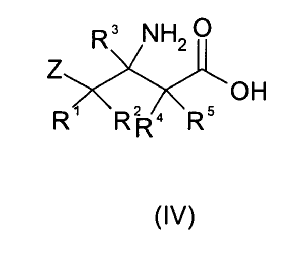 may be commercially available, known in the literature or may be conveniently synthesized using one of the methods already published and reviewed in e.g., Cole, Tetrahedron, 32, 9517 (1994), Juaristi et al., Aldrichimica Acta, 27, 3, 1994, or Juaristi, Enantioselective Synthesis of β-Amino Acids, Ed. Wiley-VCH, New York, 1997.

In particular, 3-amino-4-(2,4,5-trifluoro-phenyl)-butyric acid may be synthesized by a variety of methods as reported in the patent applications WO 2004069162, WO 2004064778, WO 2004037169, WO 2004032836 and in the articles JACS, 126, 3048 (2004) and JACS, 126, 9918 (2004).

Unless otherwise noted, all non-aqueous reactions were carried out under argon atmosphere with commercial dry solvents. Compounds were purified using flash column chromatography using Merck silica gel 60 (230-400 mesh) or reverse phase preparative HPLC using a Reprosil-Pur ODS3, 5 µm, 20 x 125 mm column with Shimadzu LC8A-Pump and SPD-10Avp UV/Vis diode array detector. The ¹H-NMR spectra were recorded on a Varian VXR-S (300 MHz for ¹H-NMR) using d₆-dimethylsulfoxide as solvent; chemical shifts are reported in ppm relative to tetramethylsilane.

Analytical LC/MS was performed using Reprosil-Pur ODS3, 5 µM, 1 x 60 mm columns with a linear gradient from 5% to 95% acetonitrile in water (0.1 % TFA or formic acid) at a flow rate of 250 µL/min; retention times are given in minutes. Methods are: (I) runs on a LC10Advp-Pump (Shimadzu) with SPD-M10Avp UV/Vis diode array detector and QP2010 MS-detector in ESI+ modus with UV-detection at 214, 254 and 275 nm, 10 min. linear gradient; (II) idem but 5 min. linear gradient; (III) runs on a LC10Advp-Pump (Shimadzu) with SPD-10Avp dual wavelength UV-detector and QP2010 MS-detector in ESI+ modus with UV-detection at 214 and 254 nm, 10 min. linear gradient; (IV) idem but 5 min. linear gradient; (V) runs on a LC10Advp-Pump (Shimadzu) with SPD-M10Avp UV/Vis diode array detector and QP2010 MS-detector in ESI+ modus with UV-detection at 220 nm, 8 min. with a linear gradient from 1% to 30% acetonitrile in water (0.1 % TFA or formic acid), then 4 min at 99%.

### General procedure for making compounds of the invention

In general, compounds having the formula (I) 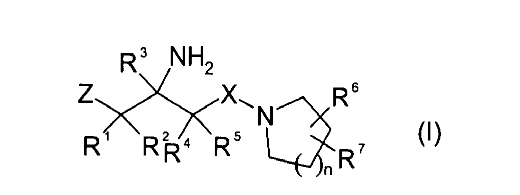 wherein the variables have the above described meanings, may be prepared using standard peptide coupling conditions, reagents and protective groups. For example, it may be possible to use 1-ethyl-3-(3-dimethylaminopropyl)carbodiimide hydrochloride (EDC) in combination with 1-hydroxybenzotriazole (HOBt) and a base (triethylamine or diisopropylethylamine) or *O*-(7-azabenzotriazol-1-yl)-N,N,N',N'-tetramethyluronium hexafluorophosphate (HATU) in the presence of a base, in solvents such as methylene chloride or *N*,*N*-dimethylformamide.

Scheme G outlines a procedure for using the amines formed according to Schemes A through F to synthesize compounds that are embodiments of the invention.

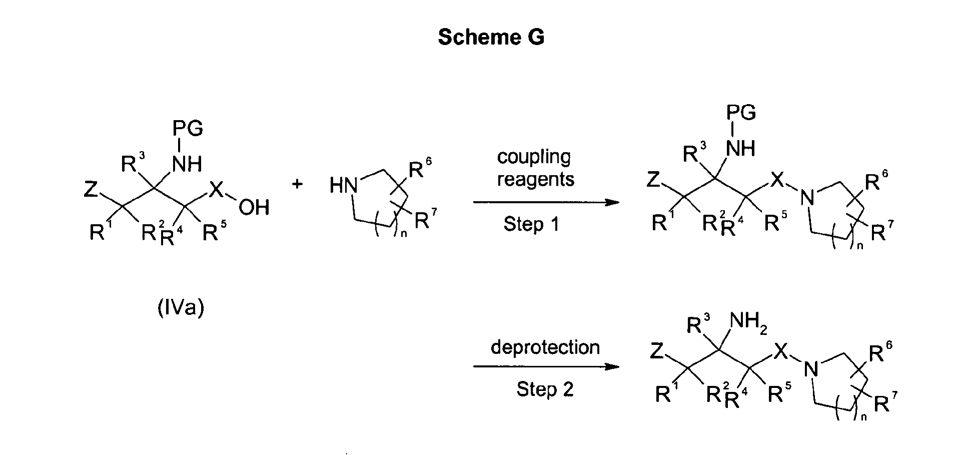

The protective group may be removed with, for example, diethylamine in dichloromethane in the case of 9-fluorenylmethoxycarbonyl or using acidic conditions (such as trifluoroacetic acid in dichloromethane or hydrochloric acid in dioxane) in the case of tert-butoxycarbonyl, as described in Protective Groups in Organic Synthesis 3rd ed., Ed. Wiley-VCH, New York; 1999.

In the case of X = S(O)₂ this may be prepared by coupling of a suitably prepared sulphonyl chloride with a suitable amine following standard sulphonylation conditions, in solvents such as dichloromethane in the presence of a base such as triethylamine or 2,6-lutidine. In the case of X = S(O) by coupling of a suitable amine with a suitable sulfinyl halide in solvents such as diethylether.

Scheme H outlines a procedure for using the amines of formula (III) and sulphonic acid chlorides of formula (IVb) to synthesize preferred compounds that are embodiments of the invention.

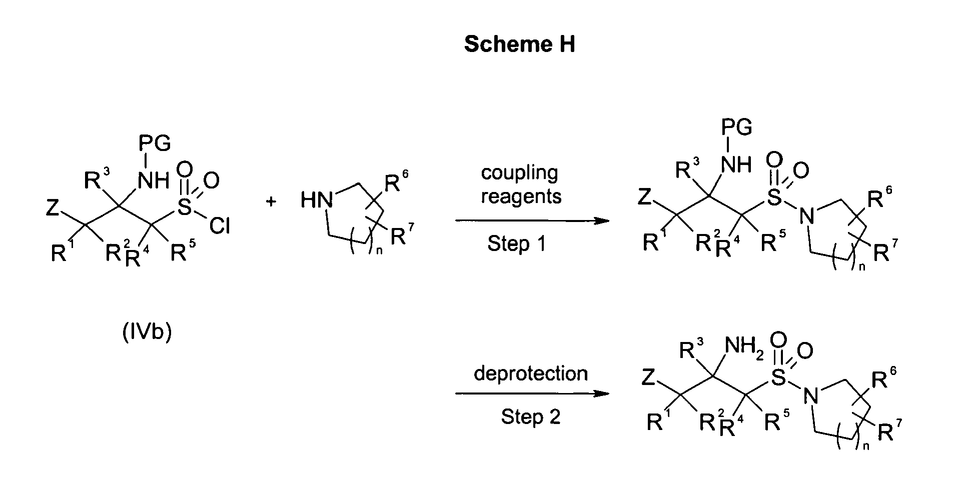

For the purification of intermediates or end products, flash chromatography on silica gel may be suitable for the free amines whereas the use of preparative HPLC leads to the isolation of the corresponding trifluoroacetic acid or formate salts.

### EXAMPLES

The following examples are provided so that the invention might be more fully understood. These examples are illustrative only and should not be construed as limiting the invention in any way.

### PREPARATIONS

The following compounds were prepared according to the following experimental procedure:

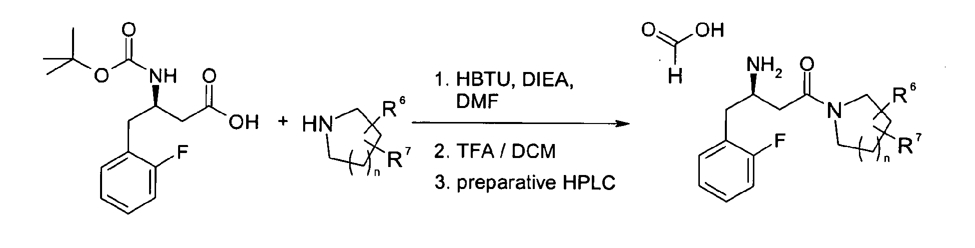

To 200 µL of a 0.6M *N*,*N*-dimethylformamide solution of *(3R)-tert*-butoxycarbonylamino-4-[2-fluoro-phenyl]-butyric acid including 1.5 eq of triethylamine in a well of a 96-microtiterplate (96-MTP), 200 µL of a 0.6M *N*,*N*-dimethylformamide solution of *O*-(benzotrialzol-1-YL)-N-N-N',N'-tetramethyluronium hexafluorophosphate (HBTU) are added. After 15 min. at room temperature, 200 µL of a 0.5M *N,N*-dimethylformamide solution of the corresponding amine and 11 µL (0.15 mmol) of *N*-methylmorpholine are added and the reaction mixture is stirred overnight at 50°C. Solvents are removed under reduced pressure, 500 µL of a solution of trifluoroacetic acid in dichloromethane (33% v/v) is added and the reaction mixture is stirred for 2 h at room temperature. After removal of the solvents under reduced pressure, 500 µL of methanol are added and the crude material is purified using preparative HPLC with a 10 min. linear gradient from 5% to 95% acetonitrile in water (0.1% TFA) to afford the title compounds.

| **Example** | **Structure** | **LC/MS** |
|---|---|---|
| 1 | 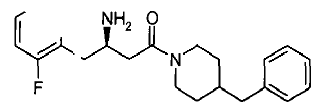 | LC/MS (V) rt 9.06, m/z 355 (M+H)⁺. |
| 2 | 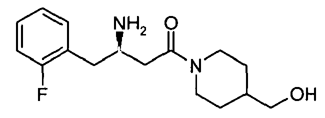 | LC/MS (V) rt 5.08, m/z 295 (MH)⁺. |
| 3 | 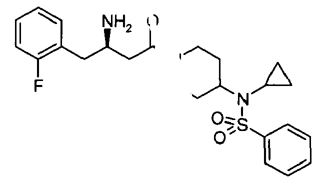 | LC/MS (V) rt 8.08, m/z 460 (M+H)⁺. |
| 4 | 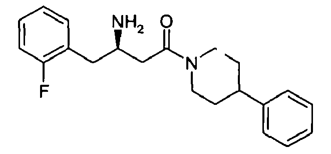 | LC/MS (V) rt 8.08, m/z 341 (M+H)⁺. |
| 5 | 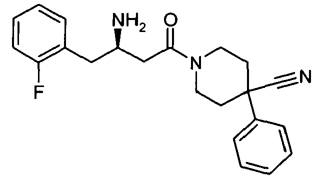 | LC/MS (V) rt 7.59, m/z 366 (M+H)⁺. |
| 6 | 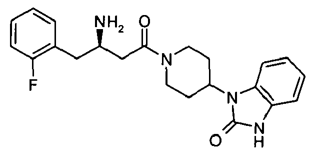 | LC/MS (V) rt 6.60, m/z 397 (M+H)⁺. |
| 7 | 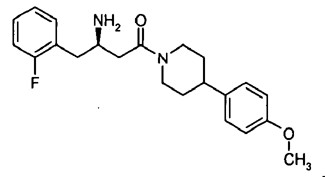 | LC/MS (V) rt 8.07, m/z 371 (M+H)⁺. |
| 8 | 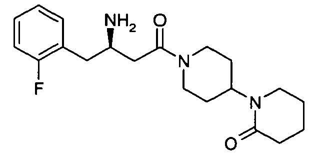 | LC/MS (V) rt 5.34, m/z 362 (M+H)⁺. |
| 9 | 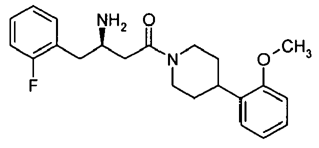 | LC/MS(V) rt 8.41, m/z 371 (M+H)⁺. |
| 10 | 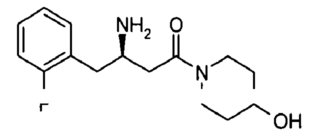 | LC/MS (V) rt 4.28, m/z 281 (M+H)⁺. |
| 11 | 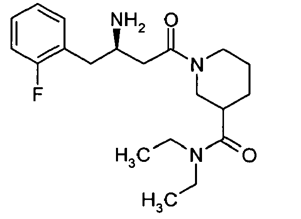 | LC/MS (V) rt 6.10, m/z 364 (M+H)⁺. |
| 12 | 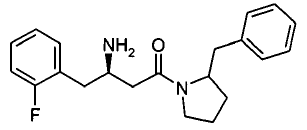 | LC/MS (V) rt 8.25, m/z 341 (M+H)⁺. |
| 13 | 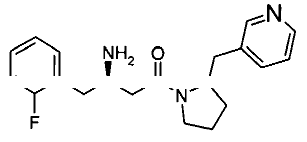 | LC/MS (V) rt 4.60, m/z 342 (M+H)⁺. |
| 14 | 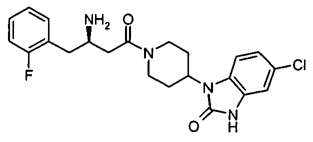 | LC/MS (V) rt 7.72, m/z 431 (M)⁺. |
| 15 | 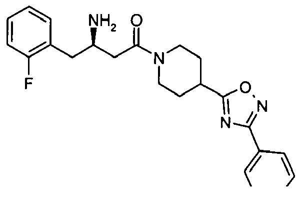 | LC/MS (V) rt 6.05, m/z 410 (M+H)⁺. |
| 16 | 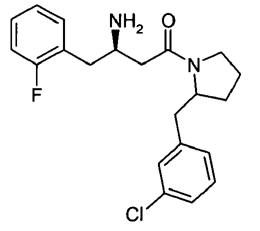 | LC/MS (V) rt 8.99, m/z 375 (M)⁺. |
| 17 | 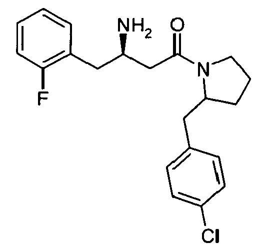 | LC/MS (V) rt 9.21, m/z 375 (M)⁺. |
| 18 | 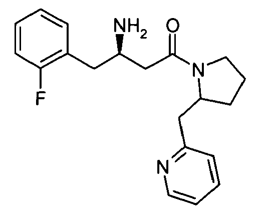 | LC/MS IV) rt 4.37, m/z 342 (M+H)⁺. |
| 19 | 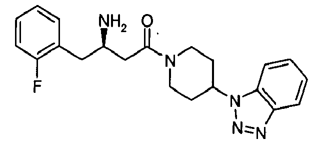 | LC/MS (V) rt 6.76, m/z 382 (M+H)⁺. |
| 20 | 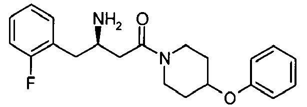 | LC/MS (V) rt 8.12, m/z 357 (M+H)⁺. |
| 21 | 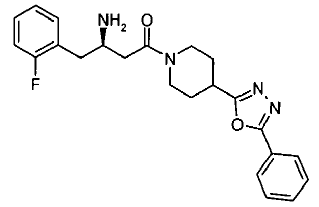 | LC/MS (V) rt 7.35, m/z 409 (M+H)⁺. |
| 22 | 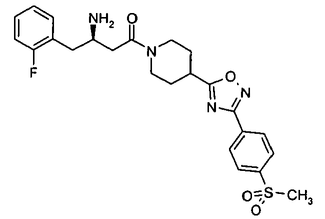 | LC/MS (V) rt 7.44, m/z 487 (M+H)⁺. |
| 23 | 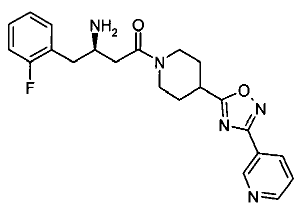 | LC/MS (V) rt 6.33, m/z 410 (M+H)⁺. |
| 24 | 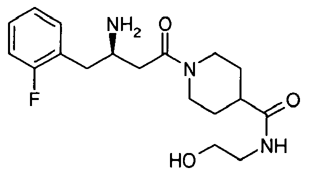 | LC/MS (V) rt 4.37, m/z 352 (M+H)⁺. |
| 25 | 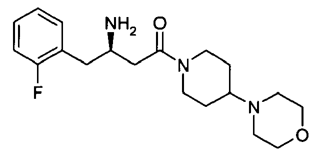 | LC/MS (V) rt 1.42, m/z 350 (M+H)⁺. |
| 26 | 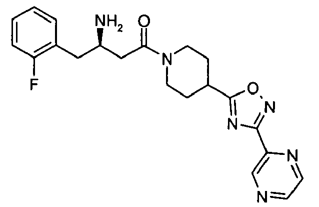 | LC/MS (V) rt 6.32, m/z 411 (M+H)⁺. |
| 27 | 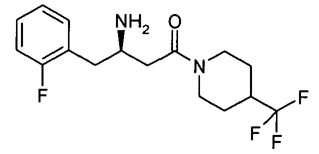 | LC/MS (V) rt 6.81, m/z 333 (M+H)⁺. |
| 28 | 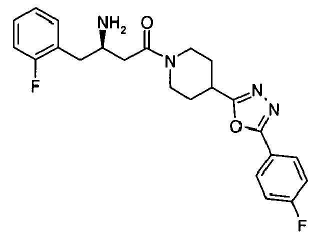 | LC/MS (V) rt 7.44, m/z 427 (M+H)⁺. |
| 29 | 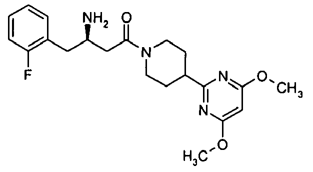 | LC/MS (V) rt 7.42, m/z 403 (M+H)⁺. |
| 30 | 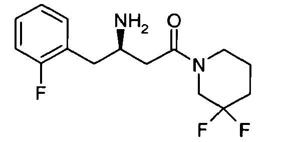 | LC/MS (V) rt 5.92, m/z 301 (M+H)⁺. |
| 31 | 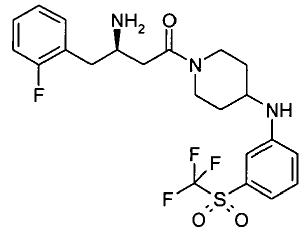 | LC/MS (V) rt 9.35, m/z 488 (M+H)⁺. |
| 32 | 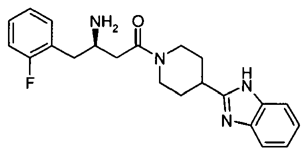 | LC/MS (V) rt 5.00, m/z 381 (M+H)⁺. |
| 33 | 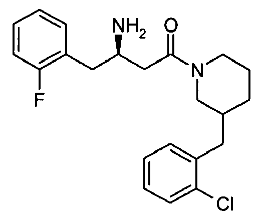 | LC/MS (V) rt 9.28, m/z 389 (M)⁺. |
| 34 | 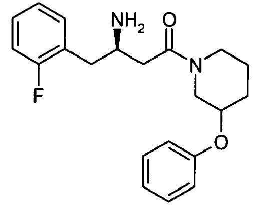 | LC/MS (V) rt 7.96, m/z 357 (M+H)⁺. |
| 35 | 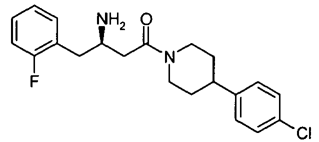 | LC/MS (V) rt 9.20, m/z 375 (M)⁺. |
| 36 | 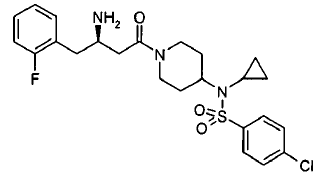 | LC/MS (V) rt 9.12, m/z 494 (M)⁺. |
| 37 | 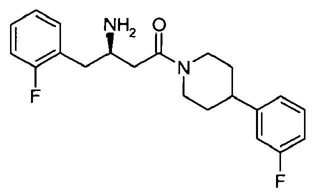 | LC/MS (V) rt 8.45, m/z 359 (M+H)⁺. |
| 38 | 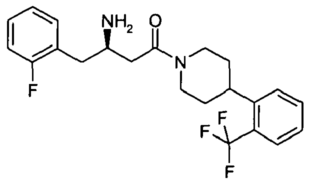 | LC/MS (V) rt 9.25, m/z 409 (M+H)⁺. |
| 39 | 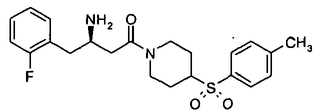 | LC/MS (V) rt 7.34, m/z 419 (M+H)⁺. |
| 40 | 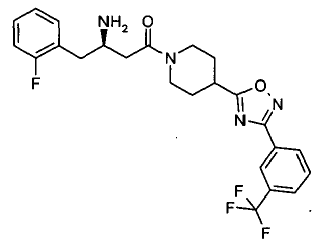 | LC/MS (V) rt 9.45, m/z 477 (M+H)⁺. |
| 41 | 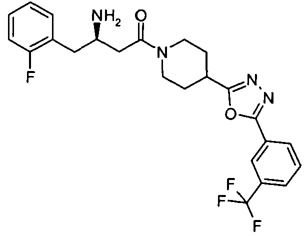 | LC/MS (V) rt 9.47, m/z 477 (M+H)⁺. |
| 42 | 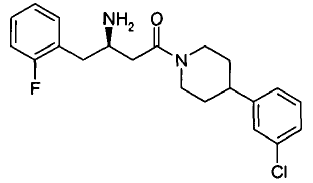 | LC/MS V) rt 9.18, m/z 375 (M)⁺. |
| 43 | 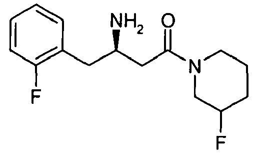 | LC/MS (V) rt 5.68, m/z 283 (M+H)⁺. |
| 44 | 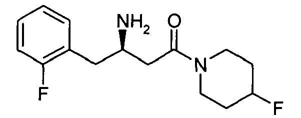 | LC/MS (V) rt 5.55, m/z 283 (M+H)⁺. |
| 45 | 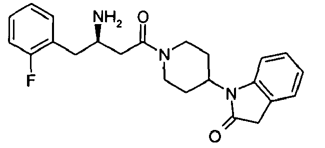 | LC/MS (V) rt 7.15, m/z 396 (M+H)⁺. |
| 46 | 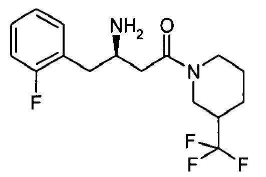 | LC/MS (V) rt 6.84, m/z 333 (M+H)⁺. |

### Example 47

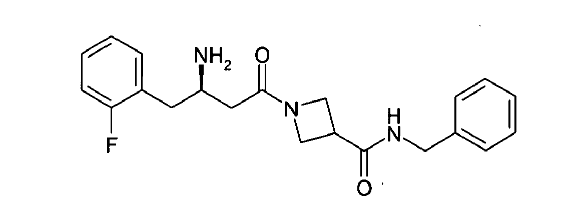

### Step 1

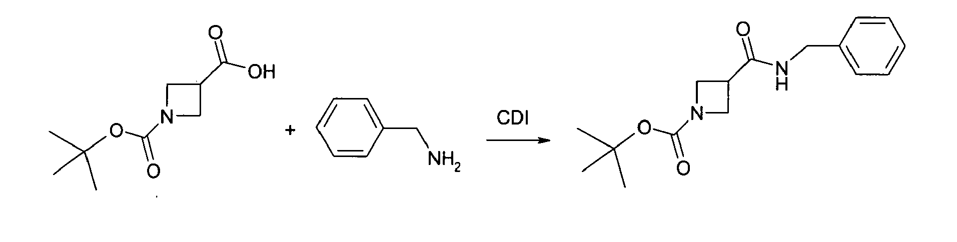

### 3-Benzylcarbamoyl-azetidine-1-carboxylic acid tert-butyl ester

In a 10 mL round-bottomed flask a mixture of 100 mg (0.497 mmole) of 1-Boc-azetidine-3-carboxylic acid and 86 mg (0.5217 mmole) 1,1'-carbonyldiimidazole (1.05 eq.) in 5 mL of dry 1,2-dichloroethane was stirred for two hours under nitrogen, then 57 µL (0.459 mmole) of benzylamine was added and the reaction mixture was stirred for 24 hours at room temperature. When the reaction was complete, the mixture was diluted with 10 mL of 1,2-dichloroethane and washed with 10 mL of 3 % citric acid, 10 mL of 10 % sodium carbonate and 10 mL of water successively, dried over magnesium sulphate, filtered and concentrated under reduced pressure affording the title compound.
¹H-NMR (CDCl₃):1.43 (s, 9H), 3.19 (m, 1H), 4.12 and 4.06 (2m, 2 x 2H), 4.46 (d, 2H), 5.8 (bs, 1 H), 7.4-7.2 (m, 5H).

### Step 2:

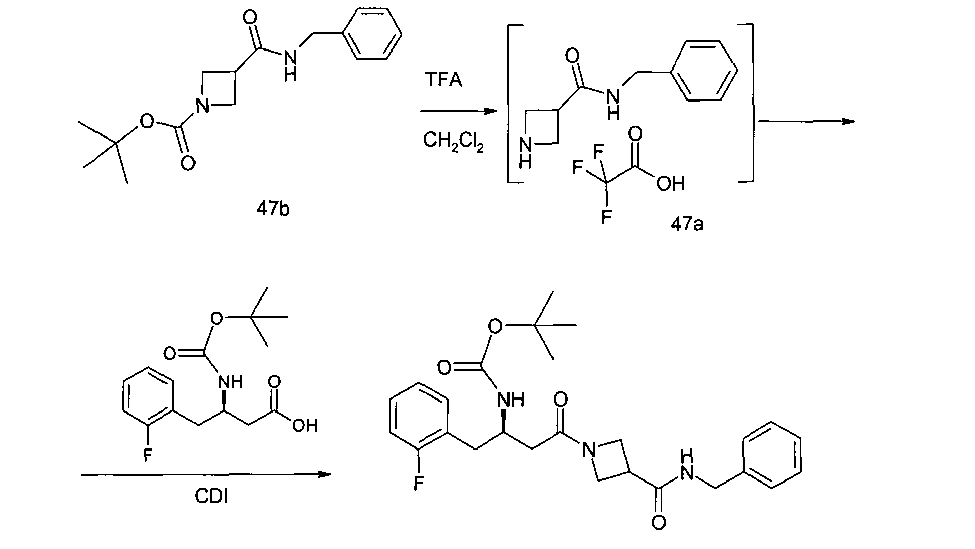

### [3-(3-Benzylcarbamoyl-azetidin-1-yl)-1 -(2-fluoro-benzyl)-3-oxo-propyl]-1-(R)-carbamic acid tert-butyl ester

In a 10 mL round-bottomed flask a mixture of 182.47 mg (0.6137 mmole) of Boc-(R)-3-amino-4-(2-fluoro-phenyl)-butyric acid and 104.5 mg (0.644 mmole) 1,1'-carbonyldiimidazole (1.05 eq.) in 8 mL of dry 1,2 dichloroethane was stirred for two hours under nitrogen. Separately, 186.8 mg (0.6137 mmole) of azetidine-3-carboxylic acid benzylamide trifluoroacetic acid salt (47a; prepared from an equivalent amount of 3-benzylcarbamoyl-azetidine-1-carboxylic acid tert-butyl ester (47b) and trifluoroacetic acid in dichloromethane, then evaporting the solvent under reduced pressure) and 117.6 µL (0.675 mmole; 1.1 eq.) of N,N-diisopropylethylamine was stirred in 8 mL of dry 1,2-dichloroethane for 15 minutes and this solution was poured into the acid and CDI reaction mixture.
Stirring was continued overnight at room temperature, then the mixture was refluxed for two days (monitored by TLC: Kieselgel Merck 5554 sheets, eluent: dichloroethane-ethanol 5:1). When the reaction was complete, the solvent was evaporated under reduced pressure and the residue was taken up in a mixture of 50 mL of dichloromethane and 50 mL of water. The water phase was extracted with 50 mL of dichloromethane and the combined organic phases were washed with 5 % of citric acid solution, 10 % of sodium-carbonate solution, water and brine successively, dried over magnesium sulphate, filtered and concentrated under reduced pressure, and the residue was subjected to preparative thin layer-chromatography on silica gel. The title compound was obtained as a white powder was obtained from hexane.
¹H-NMR (DMSO-d₆): 1.31 (s, 9H), 2.3-2.16 (m, 2H), 2.8 (m, 2H), 3.41 (m, 1H), 4.06 (m, 1H), 4.3-3.8 (2m, 2 x 2H), 4.31 (d, 2H), 6.7 (bm, 1H), 7.35-7.0 (m, 9H), 8.44 (bm, 1 H),

### Step 3

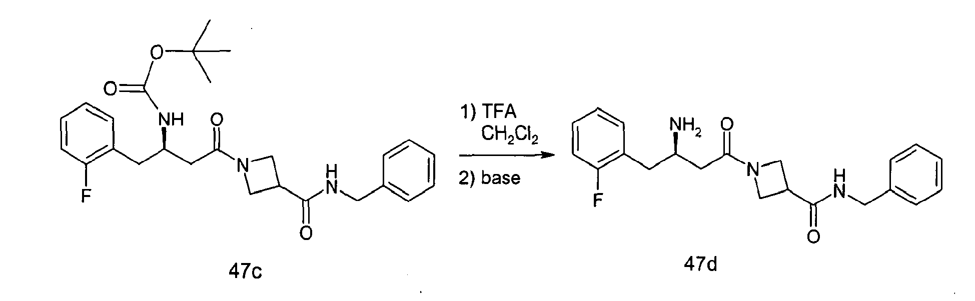

### 1-[3-(R)-amino-4-(2-fluoro-phenyl)-butyryl]-azetidine-3-carboxylic acid benzylamide

110 mg (0.2342 mmole) of [3-(3-Benzylcarbamoyl-azetidin-1-yl)-1-(2-fluoro-benzyl)-3-oxopropyl]-1-(R)-carbamic acid tert-butyl ester (47c) was dissolved with stirring in 5 mL of dry dichloromethane and 1 mL of trifluoroacetic acid was added at room temperature. The reaction mixture was allowed to stir for an hour at room temperature until the reaction was complete (monitored by TLC: Kieselgel Merck 5554 sheets, eluent: dichloroethane-ethanol 5:1). Evaporation of the solvent under reduced pressure afforded an oily residue which was taken up in 10 mL of CH₂Cl₂ and 5 mL of 5 % sodium hydroxide solution. The organic phase was washed with water and brine, dried with magnesium sulphate, and the solvent was evaporated under reduced pressure affording the title compound as a yellow oil.
¹H-NMR (CDCl₃): 1.6 (m, 2H), 2.0-2.3 (m, 2H), 2.6-2.84 (m, 2H), 3.2 (m, 1H), 3.48 (m, 1H), 4.2-4.1-4.2 and 4.36 (m, 4H), 4.44 (d, 2H), 5.9 and 6.0 (bd, 1 H), 7.0-7.37 (m, 9H).

### Example 48

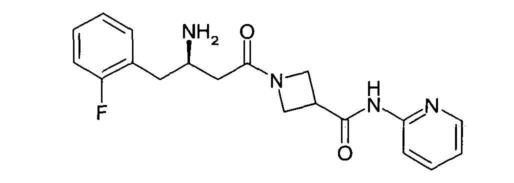

### Step 1

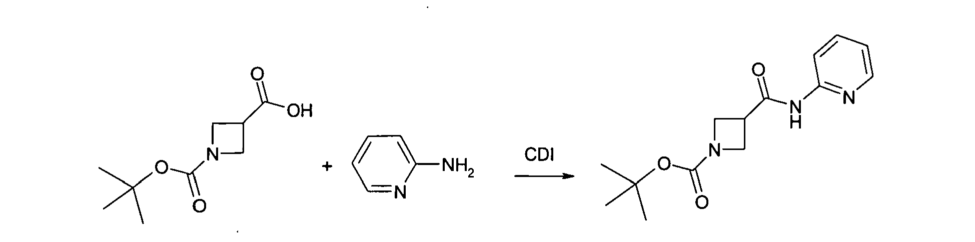

### 3-(Pyridin-2-ylcarbamoyl)-azetidine-1-carboxylic acid tert-butyl ester

Using the same preparation procedure as for example 47 (Step 1), but starting from 2-aminopyridine and refluxing for three days, then washing with 10 mL of 10% sodium carbonate and 10 mL of water, drying over magnesium sulphate, filtering, and concentrating under reduced pressure, followed by purification by chromatography (Kieselgel, eluent: chloroform : methanol 9:1) gave the title compound.
1 H-NMR (CDCl₃): 1.45 (s, 9H), 3.38 (m, 1 H), 4.10 and 4.22 (2m, 2 x 2H), 7.05 (m, 1H), 7.71 (m, 1H), 8.06 (bs, 1H), 8.22 (m, 1H), 8.3 (dd, 1H).

### Step 2:

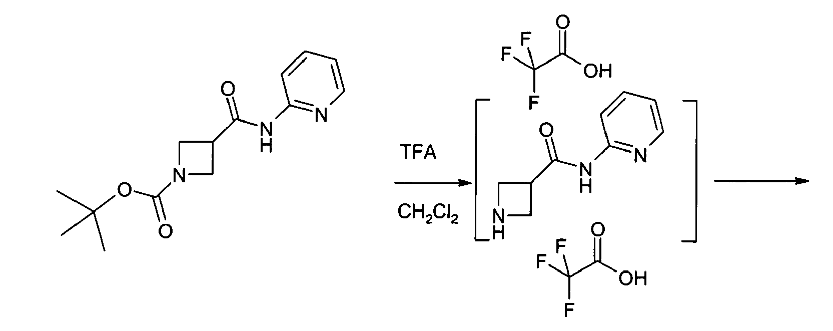

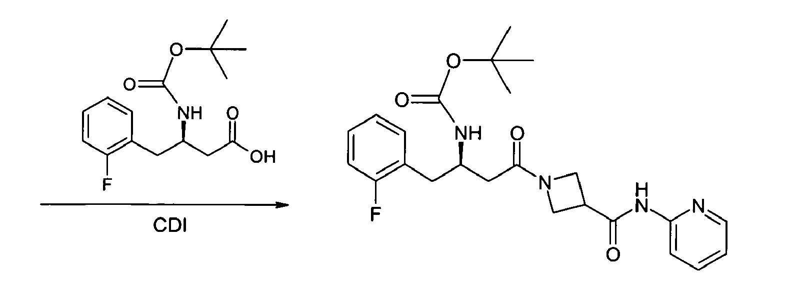

### {1-(2-Fluoro-benzyl)-3-oxo-3-[3-(pyridin-2-ylcarbamoyl)-azetidin-1-yl]-propyl}-1-(R)-carbamic acid tert-butyl ester

Using the same preparation procedure as written in case of example 47 (Step 2), but starting from azetidine-3-carboxylic acid pyridin-2-ylamide di-trifluoroacetic acid salt (prepared from an equivalent amount of 3-(pyridin-2-ylcarbamoyl)-azetidine-1-carboxylic acid tert-butyl ester and trifluoroacetic acid in dichloromethane, then evaporting the solvent under reduced pressure). A difference from the written procedure for example 47 above was that the combined organic phase was not washed with citric acid, but only with 10 % of sodium-carbonate solution, water and brine successively.
¹H-NMR (CDCl₃ + DMSO-d₆): 1.34 (s, 9H), 2.30 and 2.80 (m, 4H), 3.7 (m, 1 H), 4.00-4.35 (m, 5H), 6.5 (m, 1 H), 7.00-7.28 (m, 5H), 7.71 (m, 1H), 8.16 (m, 1 H), 8.28 (m, 1 H), 10.6 (d, 1 H).

### Step 3

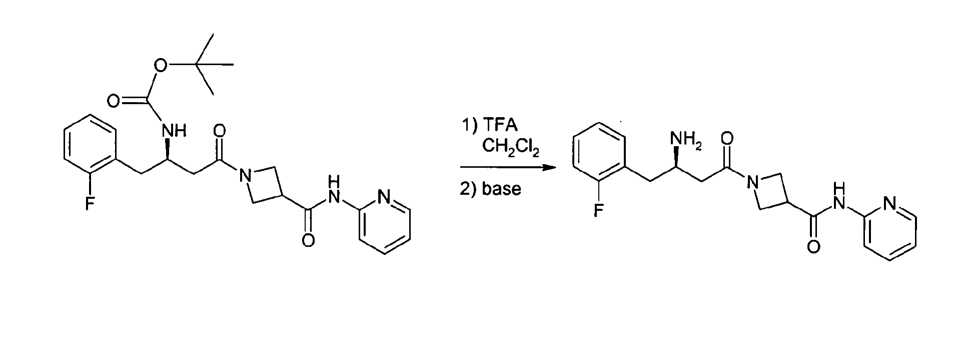

### 1-[3-(R)-amino-4-(2-fluoro-phenyl)-butyryl]-azetidine-3-carboxylic acid pyridin-2-ylamide

Using the same preparation procedure as for example 47 (Step 3), but starting from {1-(2-fluoro-benzyl)-3-oxo-3-[3-(pyridin-2-ylcarbamoyl)-azetidin-1-yl]-propyl}-1-(R)-carbamic acid tert-butyl ester (example 48, Step 2).
A white solid was obtained as the free base by treating the oil with hexane-diethylether mixture to afford the title compound.
¹H-NMR (CDCl₃): 1.6 (m, 2H), 2.05-2.32 (m, 2H), 2.62-2.85 (m, 2H), 3.4 (m, 1H), 3.58 (m, 1 H), 4.45-4.15 (m, 4H), 7.00-7.25 (m, 5H), 7.71 (ddd, 1H), 8.22 (m, 1 H), 8.24 (m, 1H), 8.3 (bs, 1 H).

### Example 49

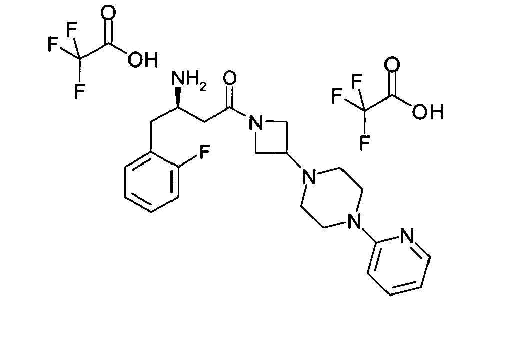

### Step 1

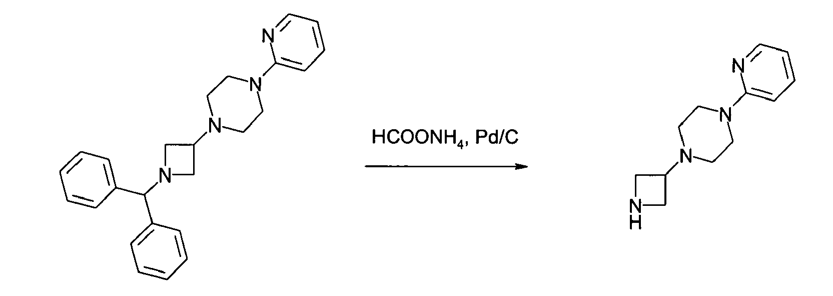

### 1-Azetidin-3-yl-4-pyridin-2-yl-piperazine

A mixture of 140 mg (0.36 mmol) of 1-(1-benzhydryl-azetidin-3-yl)-4-pyridin-2-yl-piperazine, 69 mg (1.10 mmol) of ammonium formate and 42 mg of 10% palladium on charcoal in 11 mL of ethanol are stirred at reflux overnight. After cooling to room temperature, the reaction mixture is filtered through celite and concentrated under reduced pressure. The resulting 1-azetidin-3-yl-4-pyridin-2-yl-piperazine is used in the next step without further purification.
LC/MS (IV, gradient 5-70%): rt 0.35, m/z 219 (M+H)⁺.

### Step 2

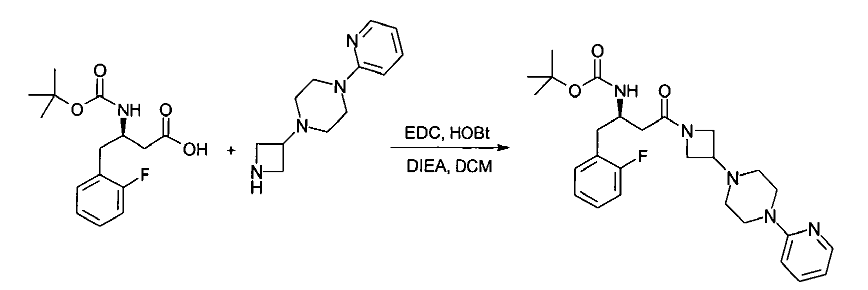

### (1R)-{1-(2-Fluoro-benzyl)-3-oxo-3-[3-(4-pyridin-2-yl-piperazin-1-yl)-azetidin-1-yl]-propyl}-carbamic acid tert-butyl ester

A mixture of 108 mg (0.36 mmol) of *(3R)*-*tert*-butoxycarbonylamino-4-[2-fluoro-phenyl]-butyric acid, 54 mg (0.40 mmol) of 1-hydroxybenzotriazole (HOBt), 77 mg (0.40 mmol) of 1-ethyl-3-(3-dimethylaminopropyl)carbodiimide hydrochloride (EDC) and 191 µL (1.09 mmol) of diisopropylethylamine (DIEA) in 7 mL of dichloromethane is stirred for 30 minutes at room temperature. Then, 19 mg (0.09 mmol) 1-azetidin-3-yl-4-pyridin-2-yl-piperazine (example 49, Step 1) in 2 mL of dichloromethane are added. The mixture is stirred overnight at room temperature. The solution is diluted with dichloromethane, washed sequentially with saturated aqueous sodium bicarbonate solution and brine, dried over sodium sulphate and concentrated under reduced pressure. The product is used in the next step without further purification.
LC/MS (IV, gradient 5-70%): rt 2.53, m/z 498 (M+H)⁺.

### Step 3

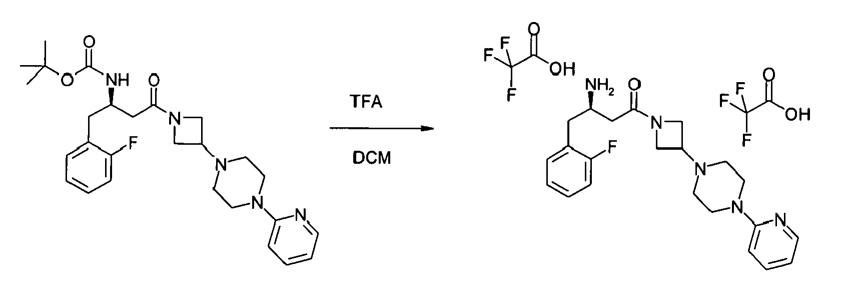

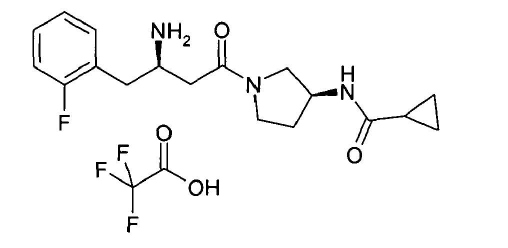

### Step 1

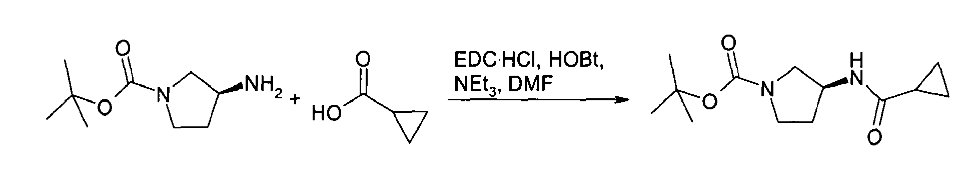

### (S)-tert-butyl 3-(cyclopropanecarboxamido)pyrrolidine-1-carboxylate

Cyclopropane carboxylic acid (48.5 mg, 0.56 mmol, 1.05 eq) is dissolved in 2 mL *N,N-*dimethylformamide and 1-hydroxybenzotriazole (76.7 mg, 0.56 mmol, 1.05 eq), triethylamine (165 µL, 1.18 mmol, 2.20 eq) and 1-ethyl-3-(3-dimethylaminopropyl)carbodiimide hydrochloride (87.5 mg, 0.56 mmol, 1.05 eq) are added subsequently. To this solution, (*S*)-3-amino-1-*N*-boc-pyrrolidine (100 mg, 0.54 mmol, 1.00 eq) is added and the resulting mixture is stirred for 12 h at room temperature. Afterwards the solution is poured into 15 mL brine and the mixture is extracted 3 times with ethyl acetate. The combined organic layers are further washed with saturated sodium hydrogencarbonate solution and brine, dried with sodium sulphate, and the solvent is evaporated under reduced pressure. The crude product is purified by flash column chromatography on silica gel with c-hexane : ethyl acetate (30:70) as eluent yielding the title compound.
LC/MS (II) rt 2.21, m/z 199, 240, 318 (M+CH₃CN+Na)⁺. (gradient 5% - 90% acetonitrile)

### Step 2

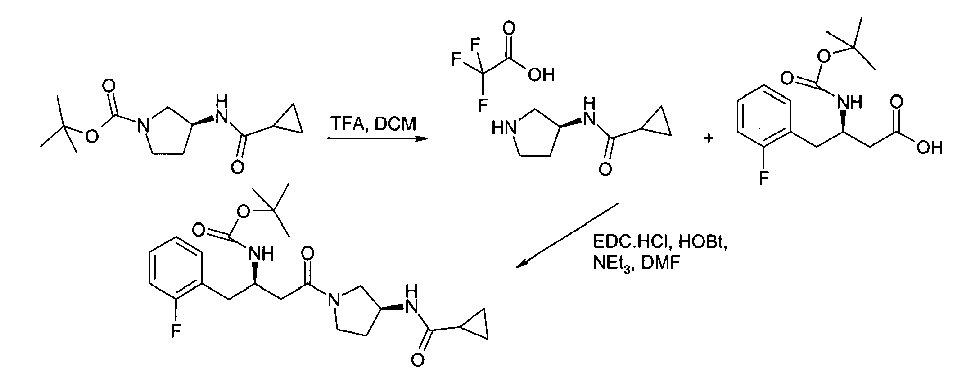

### tert-butyl-(R)-4-((S)-3-(cyclopropanecarboxamido)pyrrolidin-1-yl)-1-(2-fluorophenyl)-4-oxobutan-2-ylcarbamate

(*S*)-*tert*-butyl 3-(cyclopropanecarboxamido)pyrrolidine-1-carboxylate (96 mg, 0.38 mmol) from example 50, Step 1 is dissolved in 2 mL dichloromethane and 1 mL trifluoroacetic acid is added to the solution. The reaction mixture is stirred for 2 h at room temperature. Then 1 mL toluene is added and the solvent is evaporated. The crude (*S*)-*tert*-butyl 3-(cyclopropanecarboxamido)pyrrolidine-1-carboxylate.Trifluoroacetic acid salt is used in the peptide coupling without any further purification.
Boc-(*R*)-3-amino-4-(2-fluoro-phenyl)-butyric acid (35.2 mg, 118 µmol, 1.00 eq) is dissolved in 2 mL *N,N*-dimethylformamide and 1-hydroxybenzotriazole (24.0 mg, 178 µmol, 1.50 eq), triethylamine (59 µL, 426 µmol, 3.60 eq) and and 1-ethyl-3-(3-dimethylaminopropyl)carbodiimide hydrochloride (34.0 mg, 178 µmol, 1.50 eq) are added subsequently. To this solution, crude (*S*)-*tert*-butyl 3-(cyclopropanecarbox-amido)pyrrolidine-1-carboxylate (38.1 mg, 142 µmol, 1.20 eq) is added and the resulting mixture is stirred for 12 h at room temperature. Afterwards the solution is poured into 15 mL brine and is extracted 3 times with ethyl acetate. The combined organic layers are washed with saturated sodium hydrogencarbonate solution, water and brine, dried with sodium sulphate, and the solvent is removed under reduced pressure. The crude product is purified by flash coloum chromatography on silica gel with c-hexane : ethyl acetate (50:50) to yield the title compound.
LC/MS (II) rt 2.49, m/z 334, 434 (M+H)⁺, 456 (M+Na)⁺. (gradient 5% - 95% acetonitrile)

### Step 3

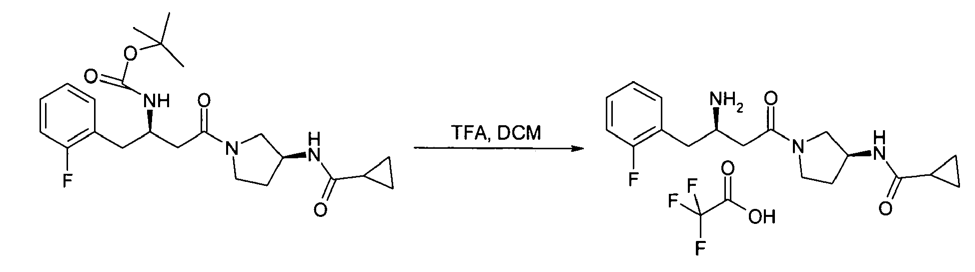

butan-2-ylcarbamate (29 mg, 66.9 µmol) from example 50, Step 2 is dissolved in 1 mL dichloromethane and treated with 0.5 mL trifluoroacetic acid. The reaction solution is stirred for 2 h at room temperature. Then 1 mL toluene is added and the solvent is evaporated under reduced pressure. The crude product is purified by preparative HPLC giving the title compound as the trifluoroacetic acid salt.
LC/MS (V) rt 5.54, m/z 334 (M+H)⁺.
¹H-NMR (300 MHz, DMSO-d₆) δ = 0.59-0.71 (m, 4H, 2 x CH₂), 1.45-1.59 (m, 1H, CH), 1.65-1.90 (m, 1 H, CH), 1.91-2.15 (m, 1H, CH), 2.80-3.21 (m, 4H, 2 x CH₂), 3.27-3.58 (m, 4H, 2 x CH₂), 3.70 (br s, 1H, CH), 4.10-4.31 (m, 1H, CH), 7.12-7.18 (m, 2H, aryl-CH), 7.28-7.35 (m, 2H, aryl-CH), 7.94 (br s, 3H, NH₃), 8.16-8.22 (dd, 1 H, NH).

### Example 51

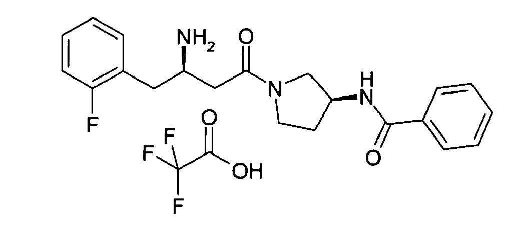

### Step 1

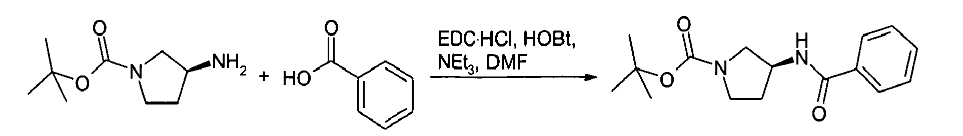

### (S)-tert-butyl 3-(benzamido)pyrrolidine-1-carboxylate

The title compound is synthesised according to Step 1, example 50. As starting materials, (*S*)-3-amino-1-*N*-boc-pyrrolidine and benzoic acid are used.
LC/MS (II) rt 2.58, m/z 235, 276, 354 (M+CH₃CN+Na)⁺. (gradient: 5% - 90% acetonitrile)

### Step 2

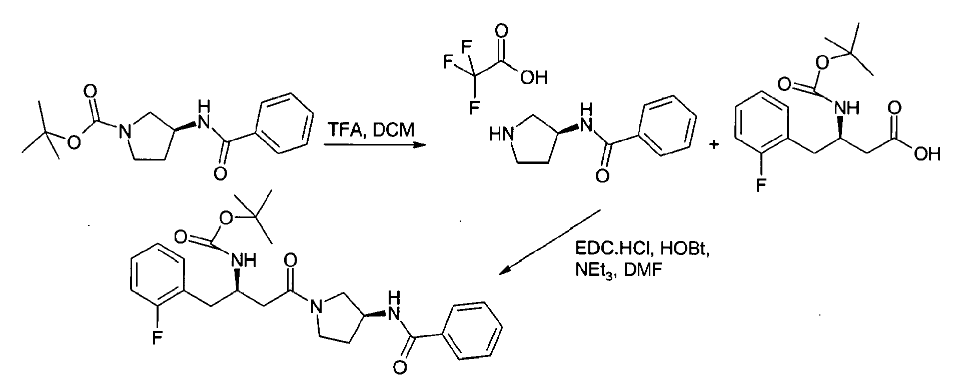

### tert-butyl-(R)-4-((S)-3-(benzamido)pyrrolidin-1-yl)-1-(2-fluorophenyl)-4-oxobutan-2-yl-carbamate

The title compound is prepared according to the procedure described for example 50, Step 2. (*S*)-*tert*-butyl 3-(benzamido)pyrrolidine-1-carboxylate and *(3R)-tert*-butoxycarbonylamino-4-[2-fluoro-phenyl]-butyric acid are used as starting material.
LC/MS (II) rt 2.80, m/z 370, 470 (M+H)⁺, 492 (M+Na)⁺. (gradient: 5% - 95% acetonitrile)

### Step 3

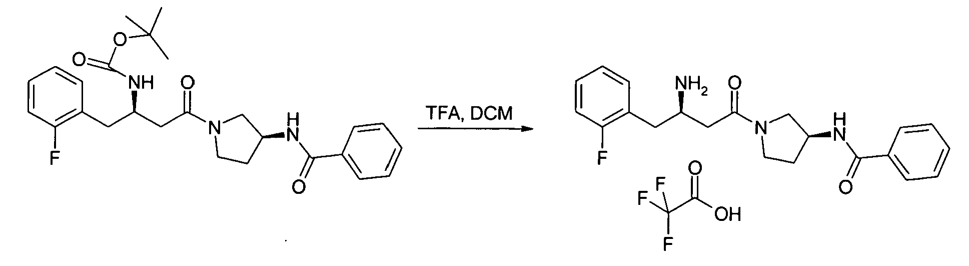

### N-((S)-1-((R)-3-amino-4-(2-fluorophenyl)butanoyl)pyrrolidin-3-yl)benzamide trifluoroacetic acid salt

To synthesise the title compound a similar procedure as described for example 50, Step 3 is used. *tert*-Butyl (*R*)-4-((*S*)-3-(benzamido)pyrrolidin-1-yl)-1-(2-fluorophenyl)-4-oxobutan-2-yl-carbamate is used as starting material.
LC/MS (V) rt 6.76, m/z 370 (M+H)⁺.
¹H-NMR (300 MHz, DMSO-d₆) δ = 1.86-2.21 (m, 2H, 2 x CH), 2.87-3.10 (m, 3H, 1 x CH, 2 x CH₂), 3.25-3.71 (m, 6H, 2 x CH; 2 x CH₂), 4.32-4.49 (m, 1H, CH), 7.11-7.18 (m, 2H, aryl-CH), 7.28-7.33 (m, 2H, aryl-CH), 7.39-7.51 (m, 4H, aryl-CH), 7.77-7.81 (m, 2H, aryl-CH), 7.97 (br s, 3H, NH₃), 8.41-8.46 (dd, 1 H, NH).

### Example 52

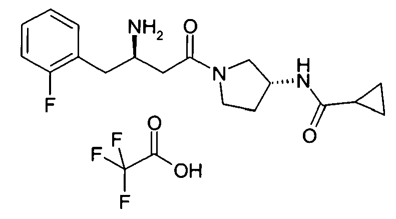

### Step 1

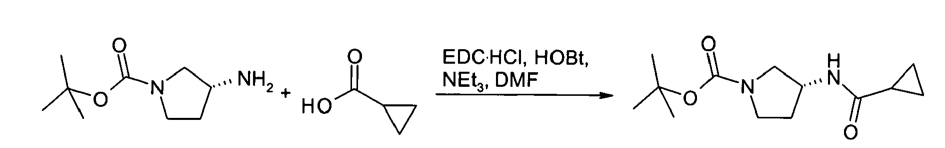

### (R)-tert-butyl 3-(cyclopropanecarboxamido)pyrrolidine-1-carboxylate

The title compound is synthesised according to Step 1, example 50. As starting materials (*R*)-3-amino-1-*N*-boc-pyrrolidine and cyclopropane carboxylic acid are used.

LC/MS (II) rt 2.17, m/z 199, 240, 318 (M+CH₃CN+Na)⁺. (gradient: 5% - 95% acetonitrile)

### Step 2

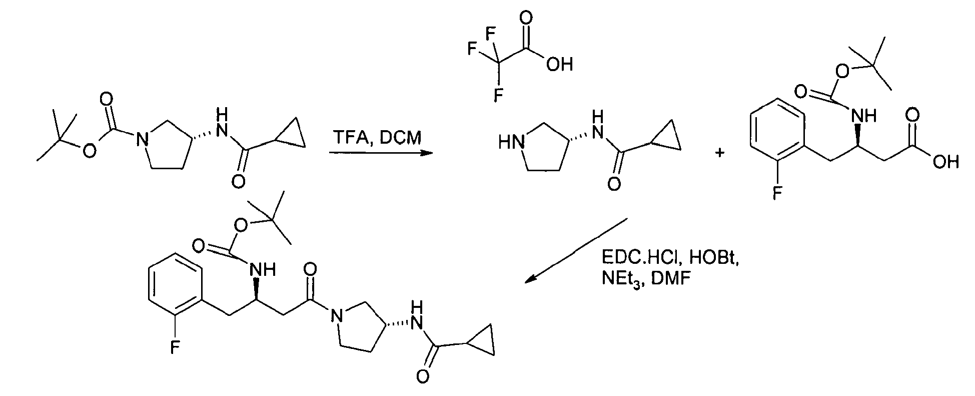

### tert-butyl-(R)-4-((R)-3-(cyclopropanecarboxamido)pyrrolidin-1-yl)-1-(2-fluorophenyl)-4-oxobutan-2-ylcarbamate

The title compound is prepared according to the procedure described for example 50, Step 2. (*R*)-*tert*-butyl-3-(cyclopropanecarboxamido)pyrrolidine-1-carboxylate and *(3R)-tert*-butoxycarbonylamino-4-[2-fluoro-phenyl]-butyric acid are used as starting material.
LC/MS (II) rt 2.53, m/z 334, 434 (M+H)⁺, 456 (M+Na)⁺. (gradient: 5% - 95% acetonitrile)

### Step 3

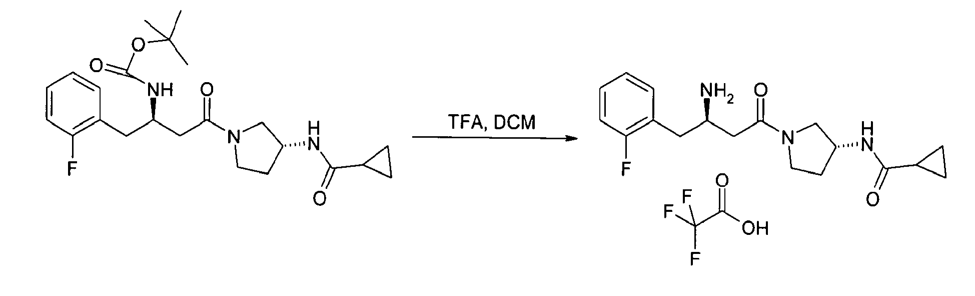

### N-((R)-1-((R)-3-amino-4-(2-fluorophenyl)butanoyl)pyrrolidin-3-yl)cyclopropane-carboxamide trifluoroacetic acid salt

To synthesise the title compound a similar procedure as described for example 50, Step 3 is used. *tert*-Butyl (*R*)-4-((*R*)-3-(cyclopropanecarboxamido)pyrrolidin-1-yl)-1-(2-fluorophenyl)-4-oxobutan-2-ylcarbamate is used as starting material.
LC/MS (V) rt 4.94, m/z 334 (M+H)⁺.
¹H-NMR (300 MHz, DMSO-d₆) δ = 0.60-0.73 (m, 4H, 2 x CH₂), 1.47-1.59 (m, 1H, CH), 1.71-1.95 (m, 1 H, CH), 1.91-2.14 (m, 1 H, CH), 2.90-3.14 (m, 4H, 2 x CH₂), 3.27-3.58 (m, 5H, 1 x CH, 2 x CH₂), 4.15-4.21 (m, 1 H, CH), 7.13-7.17 (m, 2H, aryl-CH), 7.28-7.39 (m, 2H, aryl-CH), 7.96 (br s, 3H, NH₃), 8.18-8.26 (dd, 1 H, NH).

### Example 53

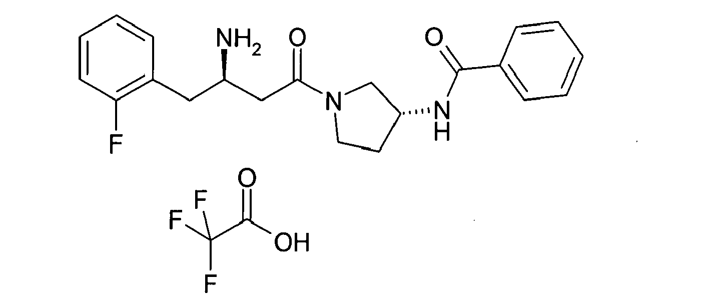

### Step 1

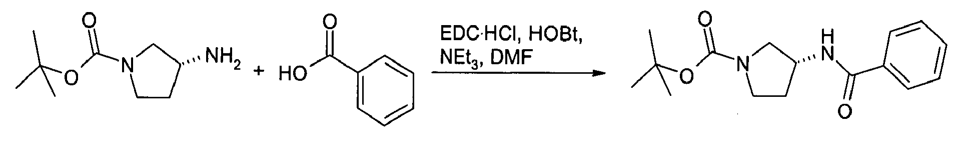

### (R)-tert-butyl 3-(benzamido)pyrrolidine-1-carboxylate

The title compound is synthesised according to Step 1, example 50. As starting materials (*R*)-3-amino-1-*N*-boc-pyrrolidine and benzoic acid are used.
LC/MS (II) rt 2.51, m/z 235, 276, 354 (M+CH₃CN+Na)⁺. (gradient: 5% - 95% acetonitrile)

### Step 2

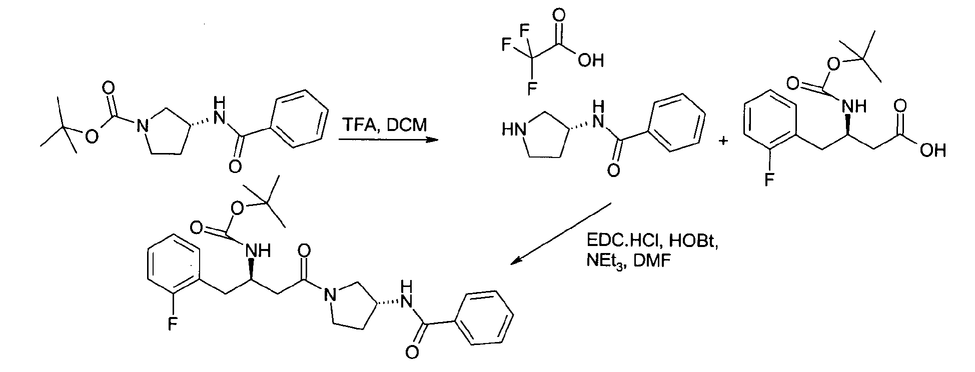

### tert-butyl-(R)-4-((R)-3-(benzamido)pyrrolidin-1-yl)-1-(2-fluorophenyl)-4-oxobutan-2-yl-carbamate

The title compound is prepared according to the procedure described for example 50, Step 2. (*R*)-*tert*-butyl 3-(benzamido)pyrrolidine-1-carboxylate and *(3R)-tert*-butoxycarbonylamino-4-[2-fluoro-phenyl]-butyric acid are used as starting material.
LC/MS (II) rt 2.75, m/z 370, 470 (M+H)⁺, 492 (M+Na)⁺. (gradient: 5% - 95% acetonitrile)

### Step 3

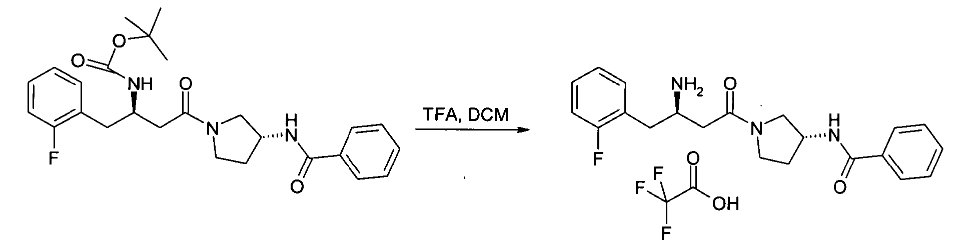

### N-((R)-1-((R)-3-amino-4-(2-fluorophenyl)butanoyl)pyrrolidin-3-yl)benzamide trifluoroacetic acid salt

To synthesise the title compound a similar procedure as described for example 50, Step 3 is used. *tert*-Butyl (*R*)-4-((*R*)-3-(benzamido)pyrrolidin-1-yl)-1-(2-fluorophenyl)-4-oxobutan-2-yl-carbamate is used as starting material.
LC/MS (V) rt 7.54, m/z 370 (M+H)⁺.
¹H-NMR (300 MHz, DMSO-d₆) δ = 1.86-2.18 (m, 2H, 2 x CH), 2.87-3.05 (m, 3H, 1 x CH, 1 x CH₂), 3.25-3.73 (m, 6H, 1 x CH; 2 x CH₂), 4.39-4.44 (m, 1 H, CH), 7.10-7.18 (m, 2H, aryl-CH), 7.28-7.32 (m, 2H, aryl-CH), 7.37-7.50 (m, 4H, aryl-CH), 7.76-7.81 (m, 2H, aryl-CH), 7.97 (br s, 3H, NH₃), 8.41-8.48 (dd, 1 H, NH).

### Example 54

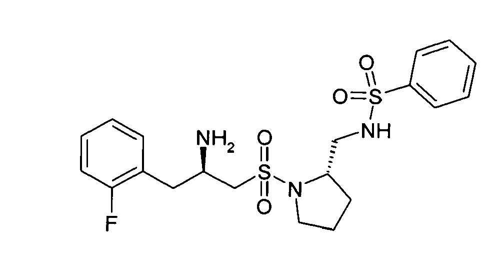

### Step 1

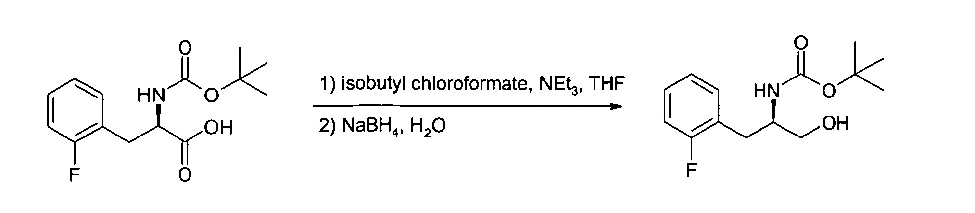

### [2(R)-(2-Fluoro-phenyl)-1-hydroxymethyl-ethyl]-carbamic acid tert-butyl ester

2.83 g (10 mmol) of 2(*R*)-*tert*-butoxycarbonylamino-3-(2-fluoro-phenyl)-propionic acid is dissolved in 10 mL of tetrahydrofuran. At 0 °C triethylamine (1.53 mL, 11 mmol) is added, followed by 1. 44 mL (11 mmol) of isobutyl chloroformate and the reaction mixture is stirred for 30 min. The precipitated solid is filtered off and the filtrate is added dropwise to a solution of 0.57 g (15 mmol) of sodium borohydride in 4 mL of water at 0 °C. After 30 min the reaction mixture is allowed to warm to room temperature and stirring is continued for additional two hours. The mixture is acidified with 4 mL of acetic acid and extracted with ethyl acetate (3 x 10 mL). The combined organic layers are washed with saturated sodium hydrogencarbonate solution, brine, dried with magnesium sulphate, filtered and evaporated under reduced pressure. The solid residue is taken up with hexane and filtered to give the title compound.
¹H-NMR (300 MHz, CDCl₃): δ = 1.38 (m, 9H), 2.3 (s, 1 H), 2.9 (m, 2H), 3.68 and 3.57 (m, 2H), 3.87 (m, 1 H), 4.8 (d, 1 H), 7.3-7.0 (m, 4H).

### Step 2

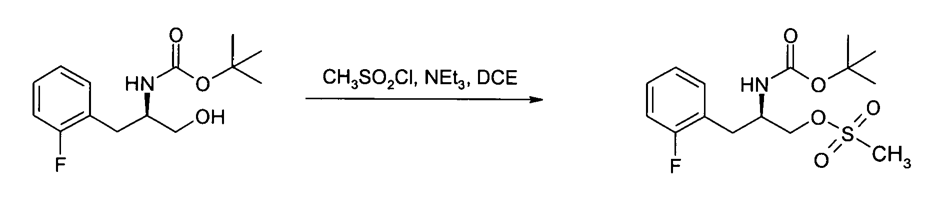

### Methanesulphonic acid 2(R)-tert-butoxycarbonylamino-3-(2-fluoro-phenyl)-propyl ester

A mixture of 1.08 g (4.00 mmol) of [2(*R*)-(2-fluoro-phenyl)-1-hydroxymethyl-ethyl]-carbamic acid *tert*-butyl ester and 620 µL (4.45 mmol) of triethylamine in 20 mL of 1,2-dichloroethane is cooled to 5 °C, and 0.49 g (330 µL, 4.42 mmol) of methanesulphonyl chloride in 5 mL of 1,2-dichloroethane is added dropwise over 30 min. The solvent is evaporated under reduced pressure, the residue is taken up in 20 mL of ethyl acetate, washed with water, 5 % saturated sodium hydrogencarbonate solution and brine, dried with magnesium sulphate, filtered and evaporated to afford a yellow powder. Recrystallization from diethylether-hexane affords a white powder as the pure mesylate compound, which is used in step 3 without further characterization.

### Step 3

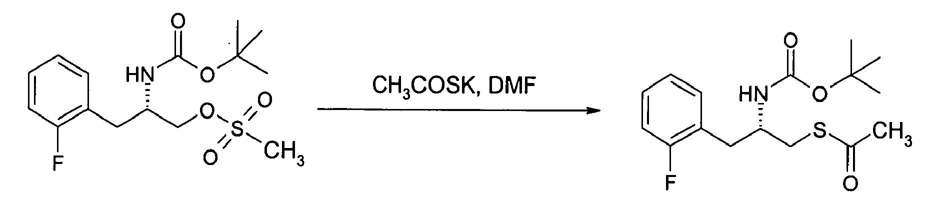

### Thioacetic acid S-[2(R)-tert-butoxvcarbonylamino-3-(2-fluoro-phenyl)-propyl] ester

The mixture of 1.1 g (3.16 mmol) of methanesulphonic acid 2(*R*)-*tert*-butoxycarbonylamino-3-(2-fluoro-phenyl)-propyl ester and 0.4 g (3.48 mmol) of potassium thioacetate is stirred in 15 mL of dry N,N-dimethylformamide for 20 hours. The reaction mixture is diluted with water, and extracted with 3 x 20 mL of ethyl acetate. The combined organic layers are washed with water, 5 % saturated sodium hydrogencarbonate solution and brine, dried with magnesium sulphate, filtered and evaporated under reduced pressure. The crude product is purified by column chromatography on silica gel with cyclohexane:ethyl acetate (80:20) as eluent. The product is further recrystalized from cyclohexane.
¹H-NMR (300 MHz, CDCl₃): δ = 1.37 (s, 9H), 2.36 (s, 3H), 2.86 (m, 2H), 3.10-3.0 (m, 2H), 4.0 (m, 1 H), 4.6 (s, 1 H), 7.3-7.0 (m, 4H).

### Step 4

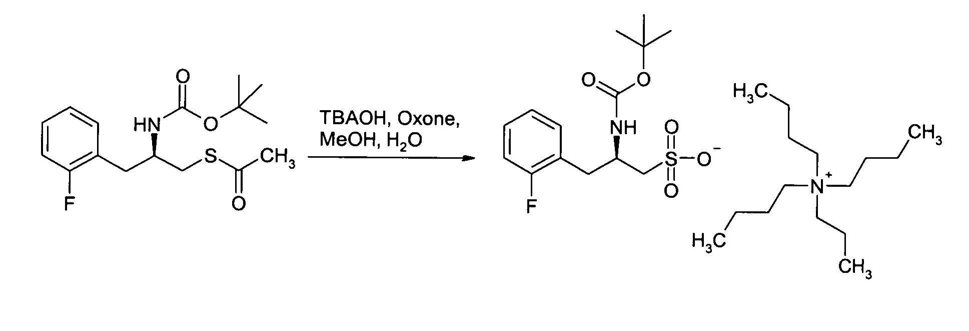

### (R)-2-tert-Butoxycarbonylamino-3-(2-fluoro-phenyl)-propane-1-sulphonate tetra-n-butyl ammonium salt

942 mg (2.61 mmol) of thioacetic acid S-[(R)-2-benzyloxycarbonylamino-3-(2-fluoro-phenyl)-propyl] ester from step 3 are dissolved in 80 mL of methanol and a solution of 4.00 g (6.50 mmol) of oxone in 80 mL of water is added. After stirring for 30 min at room temperature a solution of 4.00 mL of aqueous tetra-n-butyl ammonium hydroxide solution (40 %) in 20 mL of water is added and the resulting mixture is stirred for 12 h at room temperature. Then the bulk of the methanol is evaporated under reduced pressure, and the residue is diluted with 80 mL of water and extracted 3 times with dichloromethane. The combined organic layers are dried with sodium sulphate and the solvent is evaporated under reduced pressure to yield the title compound. The crude material is used in step 5 without further purification.

### Step 5

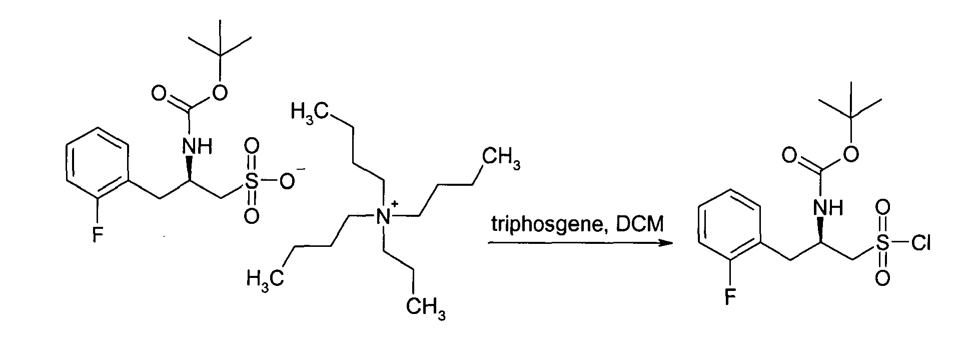

### [(R)-1-Chlorosulphonylmethyl-2-(2-fluoro-phenyl)-ethyl]-carbamic acid tert-butyl ester

78 mg (0.14 mmol) of the tetra-n-butyl ammonium salt of (R)-2-tert-butoxycarbonylamino-3-(2-fluoro-phenyl)-propane-1-sulphonic acid from step 4 are dissolved in 3 mL of dichloromethane. To this mixture a solution of 23 mg (0.09 mmol) of triphosgene in 1 mL of dichloromethane is added at 0 °C, followed by the addition of 2 drops of *N*,*N-*dimethylformamide and the reaction mixture is stirred for 1 h at 0 °C and 30 min at room temperature. Then the solvent is removed under reduced pressure and the crude product is purified by column chromatography on silica gel with dichloromethane:ethyl acetate (2:20) as eluent. The product of this reaction is used without further characterization in step 8.

### Step 6

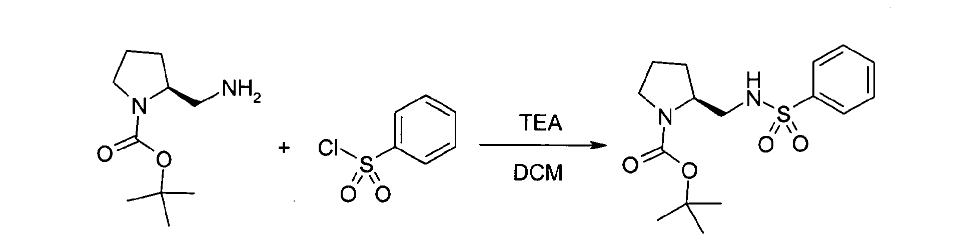

### (2S)-(Benzenesulphonylamino-methyl)-pyrrolidine-1-carboxylic acid tert-butyl ester

To a solution of 150 mg (0.75 mmol) of *(2S)*-aminomethyl-pyrrolidine-1-carboxylic acid *tert-*butyl ester and 117 µL (90.0 mg, 0.90 mmol) of triethylamine in 3 mL of dichloromethane is added 62 µL (85.7 mg, 0.80 mmol) of benzenesulphonylchloride at 0 °C. The mixture is stirred for 1.5 h at room temperature and then evaporated under reduced pressure to give a crude mixture, which is taken directly to the next step.
LC/MS (I) rt 4.34, m/z 241 (M+H-Boc)⁺.

### Step 7

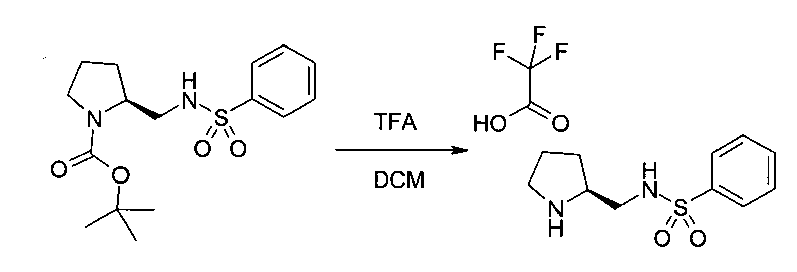

### N-Pyrrolidin-(2S)-ylmethyl-benzensulfonamide. Trifluoroacetic acid salt

A solution of 231 mg (0.47 mmol) of *(2S)*-(benzenesulphonylamino-methyl)-pyrrolidine-1-carboxylic acid *tert*-butyl ester (Step 6) in 1.5 mL of dichloromethane and 0.5 mL of trifluoroacetic acid is stirred at room temperature for 2 h and then evaporated under reduced pressure. The oily product is diluted in 5 mL of dichloromethane and filtered through aluminium oxide (eluent: 0% to 10% methanol in dichloromethane). The collected fractions are concentrated to give the title compound.
LC/MS (I) rt 2.11, m/z 241 (M+H)⁺.
¹H-NMR (300MHz, DMSO-d₆) δ (ppm) =1.53-1.65 (m, 1 H), 1.81-2.05 (m, 2H), 2.91-3.16 (m, 5H), 3.50-3.55 (m, 1 H), 7.27-7.29 (m, 1 H), 7.58-7.60 (m, 2H), 7.78-7.80 (m, 2H), 7.99 (t, J=7.6 Hz, 1 H), 9.15 (bs, 1 H).

### Step 8

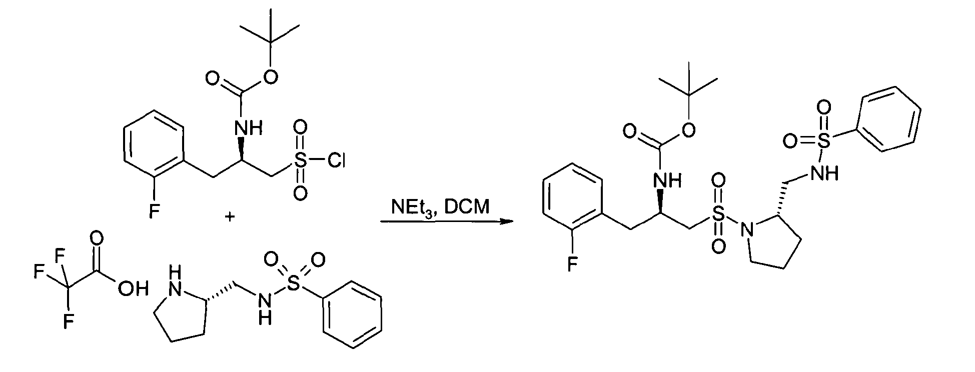

### [(R)-1-[(S)-2-(Benzenesulphonylamino-methyl)-pyrrolidine-1-sulphonylmethyl]-2-(2-fluoro-phenyl)-ethyl]-carbamic acid tert-butyl ester

46.0 mg (0.13 mmol) of [(*R*)-1-chlorosulphonylmethyl-2-(2-fluoro-phenyl)-ethyl]-carbamic acid *tert*-butyl ester (Step 5) are dissolved in 2 mL of dichloromethane. Then 40.0 µL (0.29 mmol) of triethylamine and 51.0 mg (0.14 mmol) of the trifluoroacetate salt of N-pyrrolidin-(2S)-ylmethyl-benzensulfonamide (Step 7) are added and the resulting mixture is stirred for 12 h at room temperature. The solvents are then evaporated under reduced pressure and the crude residue is purified by flash column chromatography on silica gel with cyclohexane:ethyl acetate (30:70) as eluent.
LCMS (I) rt 2.85 min, m/z 456 (M+H-Boc)⁺, 578 (M+Na)⁺.

### Step 9

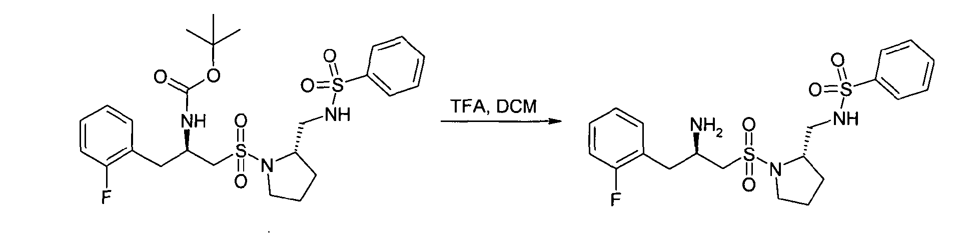

### N-{(S)-1-[(R)-2-Amino-3-(2-fluoro-phenyl)-propane-1-sulphonyl]-pyrrolidin-2-ylmethyl}-benzenesulfonamide

26.0 mg (0.05 mmol) of [(*R*)-1-[(S)-2-(benzenesulphonylamino-methyl)-pyrrolidine-1-sulphonylmethyl]-2-(2-fluoro-phenyl)-ethyl]-carbamic acid *tert*-butyl ester from step 8 are dissolved in 1.5 mL of dichloromethane and the mixture is cooled to 0 °C. Then 0.5 mL of trifluoroacetic acid is added and the reaction mixture is stirred for 1 h at 0 °C. Then 1.0 mL of toluene is added and the reaction mixture is evaporated to dryness and the residue is purified by preparative HPLC to give the title compound.
LCMS (V) rt 5.67 min, m/z 456 (M+H)⁺, 478 (M+Na)⁺.
¹H-NMR (300 MHz, DMSO-d₆) δ = 1.75-1.84 (m, 4H), 2.65-2.72 (m, 1 H), 2.78-2.85 (m, 1 H), 2.92-2.98 (m, 3H), 3.53-3.56 (m, 2H), 3.66-3.69 (m, 1 H), 7.10-7.16 (m, 2H), 7.27-7.32 (m, 2H), 7.57-7.60 (m, 3H), 7.74-7.76 (m, 2H).

### ASSAYS

Inhibition of DPP-IV peptidase activity was monitored with a continuous fluorimetric assay. This assay is based on the cleavage of the substrate Gly-Pro-AMC (Bachem) by DPP-IV, releasing free AMC. The assay is carried out in 96-well microtiterplates. In a total volume of 100 µL, compounds are preincubated with 50 pM DPP-IV employing a buffer containing 10mM Hepes, 150mM NaCl, 0.005% Tween 20 (pH 7.4). The reaction is started by the addition of 16 µM substrate and the fluorescence of liberated AMC is detected for 10 minutes at 25 °C with a fluorescence reader (BMG-Fluostar; BMG-Technologies) using an excitation wavelength of 370 nm and an emission wavelength of 450 nm. The final concentration of DMSO is 1 %. The inhibitory potential of the compounds were determined. DPP-IV activity assays were carried out with human and porcine DPP-IV (see below); both enzymes showed comparable activities-include.
Soluble human DPP-IV lacking the transmembrane anchor (Gly31-Pro766) was expressed in a recombinant YEAST-strain as Pre-Pro-alpha-mating fusion. The secreted product (rhuDPP-IV-Gly31-Pro766) was purified from fermentation broth (>90% purity) and used for inhouse screening.

The examples 1-54 show a % inhibition of about 2 µM or less.

## Claims

1. A compound of the formula (I) 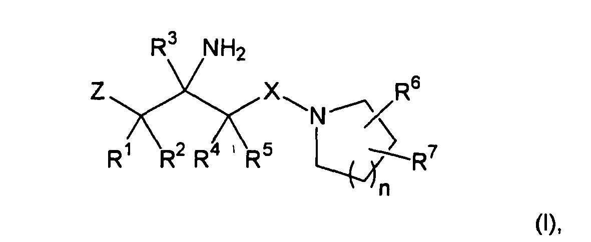 or a pharmaceutically acceptable salt or prodrug thereof, wherein
Z is selected from the group consisting of phenyl; naphthyl; indenyl; C₃₋₇ cycloalkyl; indanyl; tetralinyl; decalinyl; heterocycle; and heterobicycle, wherein Z is optionally substituted with one or more R⁸, wherein R⁸ is independently selected from the group consisting of halogen; CN; OH; NH₂; oxo (=O), where the ring is at least partially saturated; R⁹; and R¹⁰;
R⁹ is selected from the group consisting of C₁₋₆ alkyl; O-C₁₋₆ alkyl; and S-C₁₋₆ alkyl, wherein R⁹ is optionally interrupted by oxygen and wherein R⁹ is optionally substituted with one or more halogen independently selected from the group consisting of F; and Cl;
R¹⁰ is selected from the group consisting of phenyl; heterocycle; and C₃₋₇ cycloalkyl, wherein R¹⁰ is optionally substituted with one or more R¹¹, wherein R¹¹ is independently selected from the group consisting of halogen; CN; OH; NH₂; oxo (=O), where the ring is at least partially saturated; C₁₋₆ alkyl; O-C₁₋₆ alkyl; and S-C₁₋₆ alkyl;
R¹, R⁴ are independently selected from the group consisting of H; F; OH; and R^{4a};
R², R⁵ are independently selected from the group consisting of H; F; and R^{4b};
R^{4a} is independently selected from the group consisting of C₁₋₆ alkyl; and O-C₁₋₆ alkyl, wherein R^{4a} is optionally substituted with one or more halogen independently selected from the group consisting of F; and Cl;
R^{4b} is C₁₋₆ alkyl, wherein R^{4b} is optionally substituted with one or more halogen independently selected from the group consisting of F; and Cl;
R³ is selected from the group consisting of H; and C₁₋₆ alkyl;
Optionally one or more pairs of R¹, R², R³, R⁴, R⁵ independently selected from the group consisting of R¹/R²; R²/R³; R³/R⁴; and R⁴/R⁵ form a C₃₋₇ cycloalkyl ring, which is optionally substituted with one or more of R¹², wherein R¹² is independently selected from the group consisting of F; Cl; and OH;
n is 0, 1 or 2;
X is selected from the group consisting of S(O); S(O)₂; C(O); and C(R¹³R¹⁴);
R¹³, R¹⁴ are independently selected from the group consisting of H; F; C₁₋₆ alkyl; R¹⁵; and R¹⁶;
Optionally one or both pairs of R⁵, R¹³, R¹⁴ selected from the group consisting of R⁵/R¹³; and R¹³/R¹⁴ form a C₃₋₇ cycloalkyl ring, which is optionally substituted with one or more R¹⁷, wherein R¹⁷ is independently selected from the group consisting of F; Cl; and OH;
R¹⁵ is selected from the group consisting of phenyl; naphthyl; and indenyl, wherein R¹⁵ is optionally substituted with one or more R¹⁸, wherein R¹⁸ is independently selected from the group consisting of R¹⁹; R²⁰; halogen; CN; COOH; OH; C(O)NH₂; S(O)₂NH₂; S(O)NH₂; C₁₋₆ alkyl; O-C₁₋₆ alkyl; S-C₁₋₆ alkyl; COO-C₁₋₆ alkyl; OC(O)-C₁₋₆ alkyl; C(O)N(R²¹)-C₁₋₆ alkyl; S(O)₂N(R²¹)-C₁₋₆ alkyl; S(O)N(R²¹)-C₁₋₆ alkyl; S(O)₂-C₁₋₆ alkyl; S(O)-C₁₋₆ alkyl; N(R²¹)S(O)₂₋C₁₋₆ alkyl; and N(R²¹)S(O)-C₁₋₆ alkyl, wherein each C₁₋₆ alkyl is optionally substituted with one or more halogen independently selected from the group consisting of F; and Cl;
R¹⁶ is selected from the group consisting of heterocycle; heterobicycle; C₃₋₇ cycloalkyl; indanyl; tertralinyl; and decalinyl, wherein R¹⁶ is optionally substituted with one or more R²², wherein R²² is independently selected from the group consisting of R¹⁹; R²⁰; halogen; CN; OH; oxo (=O), where the ring is at least partially saturated; NH₂; COOH; C(O)NH₂; S(O)₂NH₂; S(O)NH₂; C₁₋₆ alkyl; O-C₁₋₆ alkyl; S-C₁₋₆ alkyl; N(R²³)-C₁₋₆ alkyl; COO-C₁₋₆ alkyl; OC(O)-C₁₋₆ alkyl; C(O)N(R²³)- C₁₋₆ alkyl; N(R²³)-C(O)-C₁₋₆ alkyl; S(O)₂N(R²³)-C₁₋₆ alkyl; S(O)N(R²³)-C₁₋₆ alkyl; S(O)₂-C₁₋₆ alkyl; S(O)-C₁₋₆ alkyl; N(R²³)S(O)₂-C₁₋₆ alkyl; and N(R²³)S(O)-C₁₋₆ alkyl, wherein each C₁₋₆ alkyl is optionally substituted with one or more halogen independently selected from the group consisting of F; and Cl;
R¹⁹ is selected from the group consisting of phenyl; and naphthyl, wherein R¹⁹ is optionally substituted with one or more R²⁴, wherein R²⁴ is independently selected from the group consisting of halogen; CN; COOH; OH; C(O)NH₂; S(O)₂NH₂; S(O)NH₂; C₁₋₆ alkyl; O-C₁₋₆ alkyl; S-C₁₋₆ alkyl; COO-C₁₋₆ alkyl; OC(O)-C₁₋₆ alkyl; C(O)N(R²⁵)-C₁₋₆ alkyl; S(O)₂N(R²⁵)-C₁₋₆ alkyl; S(O)N(R²⁵)-C₁₋₆ alkyl; S(O)₂-C₁₋₆ alkyl; S(O)-C₁₋₆ alkyl; N(R²⁵)S(O)₂-C₁₋₆ alkyl; and N(R²⁵)S(O) -C₁₋₆ alkyl, wherein each C₁₋₆ alkyl is optionally substituted with one or more halogen independently selected from the group consisting of F; and Cl;
R²⁰ is selected from the group consisting of heterocycle; heterobicycle; and C₃₋₇ cycloalkyl; wherein R²⁰ is optionally substituted with one or more R²⁶, wherein R²⁶ is independently selected from the group consisting of halogen; CN; OH; oxo (=O), where the ring is at least partially saturated; NH₂; COOH; C(O)NH₂; S(O)₂NH₂; S(O)NH₂; C₁₋₆ alkyl; O-C₁₋₆ alkyl; S-C₁₋₆ alkyl; N(R²⁷)-C₁₋₆ alkyl; COO-C₁₋₆ alkyl; OC(O)-C₁₋₆ alkyl; C(O)N(R²⁷)- C₁₋₆ alkyl; N(R²⁷)-C(O)-C₁₋₆ alkyl; S(O)₂N(R²⁷)-C₁₋₆ alkyl; S(O)N(R²⁷)-C₁₋₆ alkyl; S(O)₂-C₁₋₆ alkyl; S(O)-C₁₋₆ alkyl; N(R²⁷)S(O)₂-C₁₋₆ alkyl; and N(R²⁷)S(O)-C₁₋₆ alkyl wherein each C₁₋₆ alkyl is optionally substituted with one or more halogen independently selected from the group consisting of F; and Cl;
R²¹, R²³, R²⁵, R²⁷ are independently selected from the group consisting of H; and C₁₋₆alkyl, which is optionally substituted with one or more of R²⁸, wherein R²⁸ is independently selected from the group consisting of F; Cl and OH;
R⁶ is selected from the group consisting of
(1) halogen; CN; OH; NH₂; COOH; C(O)NH₂; S(O)₂NH₂; S(O)NH₂; C₁₋₆ alkyl; O-C₁₋₆ alkyl; N(R^{32a})-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆ alkyl-T³; C(O)N(R^{32a})-T³; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32B})S(O)-C₁₋₆ alkyl; C₁₋₆ alkyl-T³; OT³; wherein each C₁₋₆ alkyl is optionally substituted with one or more OH or halogen independently selected from the group consisting of F; and Cl;
with the proviso that when X is C(O), R⁶ is not (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂C₁₋₆ alkyl; (CH₂)N(R^{32a})S(O)-C₁₋₆ alkyl;
(2) phenyl; naphthyl; and indenyl; which are each optionally substituted with one or more R³⁸; wherein R³⁸ is independently selected from the group consisting of halogen; CN; R³⁹; COOH; OH; C(O)NH₂; S(O)₂NH₂; S(O)NH₂; COOT³; OT³; ST³; C(O)N(R⁴⁰)T³; S(O)₂N(R⁴⁰)T³; S(O)N(R⁴⁰)T³ and T³;
(3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of halogen; CN; R⁴²; OH; oxo (=O), where the ring is at least partially saturated; NH₂; COOH; C(O)NH₂; S(O)₂NH₂; S(O)NH₂; COOT³; OT³; C(O)N(R⁴³)T³; S(O)₂N(R⁴³)T³; S(O)N(R⁴³)T³; N(R⁴³)T³; and T³;
R^{32a} is selected from the group consisting of H; C₃₋₇ cycloalkyl and C₁₋₆ alkyl, which is optionally substituted with one or more halogen independently selected from the group consisting of F; and Cl;
R³⁹ is selected from the group consisting of C₁₋₆ alkyl; O-C₁₋₆ alkyl; S-C₁₋₆ alkyl; COO-C₁₋₆ alkyl; OC(O)- C₁₋₆ alkyl; C(O)N(R⁴⁴)-C₁₋₆ alkyl; S(O)₂N(R⁴⁴)-C₁₋₆ alkyl; S(O)N(R⁴⁴)-C₁₋₆ alkyl; S(O)-C₁₋₆ alkyl; S(O)₂-C₁₋₆ alkyl; N(R⁴⁴)S(O)₂-C₁₋₆ alkyl; and N(R⁴⁴)S(O) -C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³;
R⁴² is selected from the group consisting of C₁₋₆ alkyl; O-C₁₋₆ alkyl; S-C₁₋₆ alkyl; N(R⁴⁸)-C₁₋₆ alkyl; COO-C₁₋₆ alkyl; OC(O)- C₁₋₆ alkyl; C(O)N(R⁴⁸)- C₁₋₆ alkyl; N(R⁴⁸)-C(O)-C₁₋₆ alkyl; S(O)₂N(R⁴⁸)-C₁₋₆ alkyl; S(O) N(R⁴⁸)-C₁₋₆ alkyl; S(O)-C₁₋₆ alkyl; S(O)₂-C₁₋₆ alkyl; - N(R⁴⁸)S(O)₂-C₁₋₆ alkyl; and -N(R⁴⁸)S(O)-C₁₋₆ alkyl; wherein each C₁₋₆ alkyl is optionally substituted with one or more R⁴⁵, wherein R⁴⁵ is independently selected from the group consisting of F; COOR⁴⁹; C(O)N(R⁴⁹R⁵⁰); S(O)₂N(R⁴⁹R⁵⁰); S(O)N(R⁴⁹R⁵⁰); OR⁴⁹; N(R⁴⁹R⁵⁰); T³; O-T³; and N(R⁴⁹)-T³;
R⁴⁰, R⁴³, R⁴⁴, R⁴⁶, R⁴⁷, R⁴⁸, R⁴⁹, R⁵⁰ are independently selected from the group consisting of H; and C₁₋₆ alkyl;
T³ is selected from the group consisting of T⁴; and T⁵;
T⁴ is selected from the group consisting of phenyl; naphthyl; and indenyl; wherein T⁴ is optionally substituted with one or more R⁵¹, wherein R⁵¹ is independently selected from the group consisting of halogen; CN; COOR⁵²; OR⁵²; C(O)N(R⁵²R⁵³); S(O)₂N(R⁵²R⁵³); C₁₋₆ alkyl; O-C₁₋₆ alkyl; S-C₁₋₆ alkyl; COO-C₁₋₆ alkyl; OC(O)-C₁₋₆ alkyl; C(O)N(R⁵²)- C₁₋₆ alkyl; S(O)₂N(R⁵²)-C₁₋₆ alkyl; S(O)N(R⁵²)-C₁₋₆ alkyl; S(O)₂-C₁₋₆ alkyl; S(O) - C₁₋₆ alkyl; N(R⁵²)S(O)₂-C₁₋₆ alkyl; and N(R⁵²)S(O)-C₁₋₆ alkyl; wherein each C₁₋₆ alkyl is optionally substituted with one more halogen selected from the group consisting of F; and Cl;
T⁵ is selected from the group consisting of heterocycle; heterobicycle; C₃₋₇ cycloalkyl; indanyl; tetralinyl; and decalinyl; wherein T⁵ is optionally substituted with one or more R⁵⁴, wherein R⁵⁴ is independently selected from the group consisting of halogen; CN; OR⁵⁵; oxo (=O), where the ring is at least partially saturated; N(R⁵⁵R⁵⁶); COOR⁵⁵; C(O)N(R⁵⁵R⁵⁶); S(O)₂N(R⁵⁵R⁵⁶); S(O)N(R⁵⁵R⁵⁶); C₁₋₆ alkyl; O-C₁₋₆ alkyl; S-C₁₋₆ alkyl; N(R⁵⁵)-C₁₋₆ alkyl; COO-C₁₋₆ alkyl; OC(O)- C₁₋₆ alkyl; C(O)N(R⁵⁵)- C₁₋₆ alkyl; N(R⁵⁵)-C(O)-C₁₋₆ alkyl; S(O)₂N(R⁵⁵)-C₁₋₆ alkyl; S(O)N(R⁵⁵)-C₁₋₆ alkyl; S(O)₂-C₁₋₆ alkyl; S(O)-C₁₋₆ alkyl; N(R⁵⁵)S(O)₂-C₁₋₆ alkyl; and N(R⁵⁵)S(O)-C₁₋₆ alkyl; wherein each C₁₋₆ alkyl is optionally substituted with one more halogen selected from the group consisting of F; and Cl;
R⁵², R⁵³, R⁵⁵, R⁵⁶, are independently selected from the group consisting of H; and C₁₋₆ alkyl; and
R⁷ is selected from the group consisting of hydrogen, halogen; CN; OH; NH₂; COOH; C(O)NH₂; S(O)₂NH₂; S(O)NH₂; or C₁₋₆ alkyl.

2. The compound of claim 1 wherein
n = 0;
R⁶ is selected from the group consisting of
(1) halogen; CN; OH; NH₂; COOH; C(O)NH₂; S(O)₂NH₂; S(O)NH₂; C₁₋₆ alkyl; O-C₁₋₆ alkyl; N(R^{32a})-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆ alkyl-T³; C(O)N(R^{32a})-T³; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl; C₁₋₆ alkyl-T³; OT³; wherein each C₁₋₆ alkyl is optionally substituted with one or more OH or halogen independently selected from the group consisting of F; and Cl;
with the proviso that when X is C(O), R⁶ is not (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl;
(2) phenyl; naphthyl; and indenyl; which are each substituted with one or more R³⁸; wherein R³⁸ is independently selected from the group consisting of R³⁹; COOT³; OT³; ST³; C(O)N(R⁴⁰)T³; S(O)₂N(R⁴⁰)T³; S(O)N(R⁴⁰)T³ and T³;
(3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of R⁴²; COOT³; OT³; C(O)N(R⁴³)T³; S(O)₂N(R⁴³)T³; S(O)N(R⁴³)T³; N(R⁴³)T³; and T³;
R³⁹ is selected from the group consisting of S(O)-C₁₋₆ alkyl and S(O)₂-C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³;
R⁴² is N(R⁴⁸)-C(O)-C₁₋₆ alkyl wherein C₁₋₆ alkyl is optionally substituted with one or more R⁴⁵, wherein R⁴⁵ is independently selected from the group consisting of F; COOR⁴⁹; C(O)N(R⁴⁹R⁵⁰); S(O)₂N(R⁴⁹R⁵⁰); S(O)N(R⁴⁹R⁵⁰); OR⁴⁹; N(R⁴⁹R⁵⁰); T³; O-T³; and N(R⁴⁹)-T³.

3. The compound of claim 1 wherein
n = 2 and R⁶ is bonded in the 3-position;
R⁶ is selected from the group consisting of
(1) halogen; CN; OH; NH₂; COOH; C(O)NH₂; S(O)₂NH₂; S(O)NH₂; C₁₋₆ alkyl; O-C₁₋₆ alkyl; N(R^{32a})-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆alkyl-T³; C(O)N(R^{32a})-T³; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl; C₁₋₆ alkyl-T³; OT³; wherein each C₁₋₆ alkyl is optionally substituted with one or more OH or halogen independently selected from the group consisting of F; and Cl; with the proviso that when X is C(O), R⁶ is not (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl;
(2) phenyl; naphthyl; and indenyl; which are each substituted with one or more R³⁸; wherein R³⁸ is independently selected from the group consisting of R³⁹; COOT³; OT³; ST³; C(O)N(R⁴⁰)T³; S(O)₂N(R⁴⁰)T³; S(O)N(R⁴⁰)T³ and T³;
(3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of R⁴²; COOT³; OT³; C(O)N(R⁴³)T³; S(O)₂N(R⁴³)T³; S(O)N(R⁴³)T³; N(R⁴³)T³; and T³;
R³⁹ is selected from the group consisting of S(O)-C₁₋₆ alkyl and S(O)₂-C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³.
R⁴² is N(R⁴⁸)-C(O)-C₁₋₆ alkyl wherein C₁₋₆ alkyl is optionally substituted with one or more R⁴⁵, wherein R⁴⁵ is independently selected from the group consisting of F; COOR⁴⁹; C(O)N(R⁴⁹R⁵⁰); S(O)₂N(R⁴⁹R⁵⁰); S(O)N(R⁴⁹R⁵⁰); OR⁴⁹; N(R⁴⁹R⁵⁰); T³; O-T³; and N(R⁴⁹)-T³.

4. The compound of claim 1 wherein
n = 2 and R⁶ is bonded in the 2-position;
R⁶ is selected from the group consisting of
(1) halogen; OH; NH₂; COOH; C(O)NH₂; S(O)₂NH₂; S(O)NH₂; O-C₁₋₆ alkyl; N(R^{32a})-C₁₋₆ alkyl; COO-C₁₋₆ alkyl; OC(O)-C₁₋₆ alkyl; C(O)N(R^{32a})-C₁₋₆ alkyl; C(O)N(R^{32a})-C₁₋₆ alkyl-T³; C(O)N(R^{32a})-T³; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl; OT³; wherein each C₁₋₆ alkyl is optionally substituted with one or more OH or halogen independently selected from the group consisting of F; and CI;
with the proviso that when X is C(O), R⁶ is not (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl;
(2) phenyl; naphthyl; and indenyl; which are each substituted with one or more R³⁸; wherein R³⁸ is independently selected from the group consisting of R³⁹; COOT³; OT³; ST³; C(O)N(R⁴⁰)T³; S(O)₂N(R⁴⁰)T³; S(O)N(R⁴⁰)T³ and T³;
(3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of R⁴²; COOT³; OT³; C(O)N(R⁴³)T³; S(O)₂N(R⁴³)T³; S(O)N(R⁴³)T³; N(R⁴³)T³; and T³;
R³⁹ is selected from the group consisting of S(O)-C₁₋₆ alkyl and S(O)₂-C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³;
R⁴² is N(R⁴⁸)-C(O)-C₁₋₆ alkyl wherein C₁₋₆ alkyl is optionally substituted with one or more R⁴⁵, wherein R⁴⁵ is independently selected from the group consisting of F; COOR⁴⁹; C(O)N(R⁴⁹R⁵⁰); S(O)₂N(R⁴⁹R⁵⁰); S(O)N(R⁴⁹R⁵⁰); OR⁴⁹; N(R⁴⁹R⁵⁰); T³; O-T³; and N(R⁴⁹)-T³.

5. The compound of claim 1 wherein
n = 1;
R⁶ is selected from the group consisting of
(1) OH; NH₂; COOH; C(O)NH₂; S(O)₂NH₂; S(O)NH₂; O-C₁₋₆ alkyl; N(R^{32a})-C₁₋₆ alkyl; COO-C₁₋₆ alkyl; OC(O)-C₁₋₆ alkyl; N(R^{32a})-C(O)-C₁₋₆ alkyl; N(R^{32a})-C(O)-T³; S(O)₂N(R^{32a})-C₁₋₆ alkyl; S(O)N(R^{32a})-C₁₋₆ alkyl; N(R^{32a})S(O)₂T³; N(R^{32a})T³; S(O)₂-T³; S(O)₂-C₁₋₆ alkyl; S(O)-C₁₋₆ alkyl; N(R^{32a})S(O)₂-C₁₋₆ alkyl; N(R^{32a})S(O)-C₁₋₆ alkyl; C₁₋₆ alkyl-T³; (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl; OT³; wherein each C₁₋₆ alkyl is optionally substituted with one or more OH or halogen independently selected from the group consisting of F; and Cl;
with the proviso that when X is C(O), R⁶ is not (CH₂)NH₂; (CH₂)COOH; (CH₂)C(O)NH₂; (CH₂)S(O)₂NH₂; (CH₂)S(O)NH₂; (CH₂)O-C₁₋₆ alkyl; (CH₂)N(R^{32a})-C₁₋₆ alkyl; (CH₂)COO-C₁₋₆ alkyl; (CH₂)OC(O)-C₁₋₆ alkyl; (CH₂)C(O)N(R^{32a})-C₁₋₆ alkyl; (CH₂)C(O)N(R^{32a})-C₁₋₆ alkyl-T³; (CH₂)C(O)N(R^{32a})-T³; (CH₂)N(R^{32a})-C(O)-C₁₋₆ alkyl; (CH₂)N(R^{32a})-C(O)-T³; (CH₂)S(O)₂N(R^{32a})-C₁₋₆ alkyl; (CH₂)S(O)N(R^{32a})-C₁₋₆ alkyl; (CH₂)N(R^{32a})S(O)₂T³; (CH₂)N(R^{32a})T³; S(O)₂-T³; (CH₂)S(O)₂-C₁₋₆ alkyl; (CH₂)S(O)-C₁₋₆ alkyl; (CH₂)N(R^{32a})S(O)₂-C₁₋₆ alkyl; (CH₂)N(R^{32a})S(O)-C₁₋₆ alkyl;
(2) phenyl; naphthyl; and indenyl; which are each substituted with one or more R³⁸; wherein R³⁸ is independently selected from the group consisting of R³⁹; COOT³; OT³; ST³; C(O)N(R⁴⁰)T³; S(O)₂N(R⁴⁰)T³; S(O)N(R⁴⁰)T³ and T³;
(3) C₃₋₇ cycloalkyl; indanyl; tetralinyl; decalinyl; heterocycle; and heterobicycle; which are each optionally substituted with one or more R⁴¹, wherein R⁴¹ is independently selected from the group consisting of R⁴²; COOT³; OT³; C(O)N(R⁴³)T³; S(O)₂N(R⁴³)T³; S(O)N(R⁴³)T³; N(R⁴³)T³; and T³;
R³⁹ is selected from the group consisting of S(O)-C₁₋₆ alkyl and S(O)₂-C₁₋₆ alkyl; wherein each C₁₋₆ alkyl is optionally substituted with one more R⁴⁵, wherein R⁴⁵ is independently selected from the group consisting of F; COOR⁴⁶; C(O)N(R⁴⁶R⁴⁷); S(O)₂N(R⁴⁶R⁴⁷); OR⁴⁶; N(R⁴⁶R⁴⁷); T³; O-T³; and N(R⁴⁶)-T³;
R⁴² is N(R⁴⁸)-C(O)-C₁₋₆ alkyl wherein C₁₋₆ alkyl is optionally substituted with one or more R⁴⁵, wherein R⁴⁵ is independently selected from the group consisting of F; COOR⁴⁹; C(O)N(R⁴⁹R⁵⁰); S(O)₂N(R⁴⁹R⁵⁰); S(Q)N(R⁴⁹R⁵⁰); OR⁴⁹; N(R⁴⁹R⁵⁰); T³; O-T³; and N(R⁴⁹)-T³.

6. The compound of claim 1 wherein
n = 2 and R⁶ is bonded in the 4-position.

7. The compound according to any one of the preceding claims of formula (Ia) 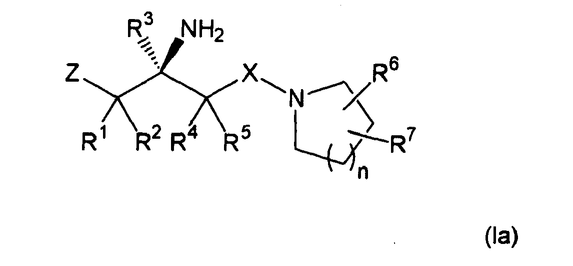 or a pharmaceutically acceptable salt thereof, wherein Z, R¹-R⁷, X and n have the meaning as indicated in claims 1 to 6.

8. The compound according to any one of the preceding claims, wherein Z is phenyl or heterocycle.

9. The compound according to any one of the preceding claims, wherein Z is optionally substituted with 1, 2 or 3 R⁸, which are the same or different.

10. The compound according to any one of the preceding claims, wherein R⁸ is selected from the group consisting of Cl; F; CN; CH₃; and OCH₃.

11. The compound according to any one of the preceding claims, wherein Z is 2-fluoro-phenyl or 2,4,5-trifluoro-phenyl.

12. The compound according to any one of the preceding claims, wherein R¹, R⁴ are independently selected from the group consisting of H; F; OH; CH₃; and OCH₃.

13. The compound according to any one of the preceding claims, wherein R², R⁵ are independently selected from the group consisting of H; F; and CH₃.

14. The compound according to any one of the preceding claims, wherein R¹, R², R⁴, R⁵ are H.

15. The compound according to any one of the preceding claims, wherein R³ is H.

16. The compound according to any one of the preceding claims, wherein X is C(O) or S(O)₂.

17. The compound according to any one of the preceding claims, wherein
n is 2 and R⁶ is bound in the 4-position, and R⁶ is selected from the group consisting of (1); (2) and/or (3)
(1) F; Cl; OH; C₁₋₂ alkyl; C(O)NH-C₁₋₂ alkyl; N(cyclopropyl)S(O)₂-phenyl; NH-phenyl; C₁₋₂ alkyl-phenyl; C₁₋₂ alkyl-phenyl; O-phenyl; wherein each phenyl is optionally substituted with halogen; CN; OH; C₁₋₆ alkyl; O-C₁₋₆ alkyl; S(O)₂-C₁₋₆ alkyl; NHS(O)₂-C₁₋₆ alkyl; and wherein each C₁₋₂ alkyl is optionally substituted with 1, 2 or 3 F or 1 OH;
(2) phenyl which is optionally substituted with one or more R³⁸; wherein R³⁸ is independently selected from the group consisting of halogen; CN; COOH; OH; C₁₋₄ alkyl; O-C₁₋₄ alkyl; wherein each C₁₋₄ alkyl is optionally substituted with 1, 2 or 3 R⁴⁵, wherein R⁴⁵ is independently selected from the group consisting of F or OH;
(3) a heterocycle selected from a 6-membered N-containing ring which may be saturated or aromatic, containing 1 or 2 N atoms, or a 5-membered N-containing aromatic ring, containing 1 or 2 N atoms, or
a heterobicycle selected from fused N-containing rings, preferably a benzyl ring fused to a N-containing 5-membered ring which is saturated;
whereby both the heterocycle and the heterobicycle are each optionally substituted with one or more R⁴¹ is independently selected from the group consisting of
halogen, oxo (=O), where the ring is at least partially saturated;
phenyl optionally substituted with preferably halogen; C₁₋₂ alkyl; S(O)₂-C₁₋₂ alkyl; and
5- or 6-membered N-containing heterocycle optionally substituted with halogen; CN; OH; C₁₋₆ alkyl; O-C₁₋₆ alkyl; S(O)₂-C₁₋₆ alkyl; NHS(O)₂-C₁₋₆ alkyl.

18. The compound according to any one of the preceding claims, wherein
n is 0 and R⁶ is selected from the group consisting of
(1) C(O)NH-C₁₋₂ alkyl-T³; or C(O)NH-T³; wherein T³ is phenyl or a 5- or 6-membered N-containing heterocycle, wherein phenyl and the heterocycle are each independently optionally substituted with halogen; CN; OH; C₁₋₆ alkyl; O-C₁₋₆ alkyl; S(O)₂-C₁₋₆ alkyl; NHS(O)₂-C₁₋₆ alkyl;
(3) 5- or 6-membered, N-containing heterocycle which is optionally substituted with one or more R⁴¹, wherein R⁴¹ is independently selected from the group consisting of OT³; N(R⁴³)T³; and T³; whereby T³ is phenyl, pyridyl or pyrazinyl.

19. The compound according to any one of the preceding claims, wherein
n is 2 and R⁶ is bound in the 3-position, whereby R⁶ is selected from the group consisting of F; Cl; C₁₋₂ alkyl; C(O)NHC₁₋₄ alkyl; C(O)N(C₁₋₄ alkyl)C₁₋₄ alkyl;
C₁₋₄ alkyl-phenyl optionally substituted with halogen; CN; OH; C₁₋₆ alkyl; O-C₁₋₆ alkyl; S(O)₂-C₁₋₄ alkyl; NHS(O)₂-C₁₋₄ alkyl; wherein each C₁₋₄ alkyl is optionally substituted with 1 OH or 1, 2 or 3 halogen independently selected from the group consisting of F; and Cl;
O-phenyl optionally substituted with halogen; CN; OH; C₁₋₆ alkyl; O-C₁₋₆ alkyl; S(O)₂-C₁₋₄ alkyl; NHS(O)₂-C₁₋₄ alkyl; wherein each C₁₋₄ alkyl is optionally substituted with 1 OH or 1, 2 or 3 halogen independently selected from the group consisting of F; and Cl; wherein each alkyl is optionally substituted with 3 F.

20. The compound according to any one of the preceding claims, wherein
n is 1 and R⁶ is selected from the group consisting of NH-C(O)-phenyl optionally substituted with halogen; C₁₋₂ alkyl; S(O)₂-C₁₋₂ alkyl;
NH-C(O)-C₃₋₇ cycloalkyl;
CH₂-phenyl optionally substituted with halogen; C₁₋₂ alkyl; S(O)₂-C₁₋₂ alkyl; and
CH₂-5- or 6-membered N-containing heterocycle whereby the heterocycle is optionally substituted with halogen; CN; OH; C₁₋₆ alkyl; O-C₁₋₆ alkyl; S(O)₂₋C₁₋₆ alkyl; NHS(O)₂-C₁₋₆ alkyl.

21. A pharmaceutical composition comprising a compound or a pharmaceutically acceptable salt thereof according to any one of the claims 1 to 20 together with a pharmaceutically acceptable carrier.

22. A pharmaceutical composition according to claim 21, comprising one or more additional compounds or pharmaceutically acceptable salts thereof selected from the group consisting of another compound according to any one of the claims 1 to 20; another DPP-IV inhibitor; insulin sensitizers; PPAR agonists; biguanides; protein tyrosinephosphatase-IB (PTP-1B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoly dual agonists; inhibitors of cholesterol absorption; acyl CoA : cholesterol acyltransferase inhibitors; anti-oxidants; PPARo agonists; antiobesity compounds; an ileal bile acid transporter inhibitor; and anti-inflammatory agents.

23. A compound or a pharmaceutically acceptable salt thereof of any one of the claims 1 to 20 for use as a medicament.

24. Use of a compound or a pharmaceutically acceptable salt thereof of any of the claims 1 to 20 for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; anxiety; depression; retinopathy; nephropathy; neuropathy; Syndrome X; ovarian hyperandrogenism (polycystic ovarian syndrome; Type n diabetes; or growth hormone deficiency.

25. Use of a compound according to any one of the claims 1 to 20 as DPP-IV inhibitor.
